# EUROPEAN PATENT SPECIFICATION

(11) **EP 3 060 204 B1**
(45) Date of publication and mention of the grant of the patent: **24.11.2021**
(21) Application number: 14790263.9
(22) Date of filing: 21.10.2014
(51) Int. Cl.: A61K 31/18, A61P 35/00

(54) **BETA-CATENIN**
BETA-CATENIN
BÊTA-CATÉNINE

(30) Priority: 22.10.2013 GB 201318659
(43) Date of publication of application: 31.08.2016
(73) Proprietor: Ahmed, Aamir, London N12 9HS (GB); Thrasivoulou, Christopher, London E10 7HJ (GB)
(72) Inventor: AHMED, Aamir, London N12 9HS (GB); THRASIVOULOU, Christopher, London E10 7HJ (GB); MASTERS, John, London W1T 4TP (GB)
(74) Representative: Hutter, Anton
(86) International application number: PCT/GB2014/053138
(87) International publication number: WO 2015/059463

(56) References cited:
- EP-A2- 0 123 850
- WO-A1-2006/039806
- WO-A2-2011/058508
- Kobayashi: "Nuclear translocation of beta-catenin in colorectal cancer", , 21 April 2000 (2000-04-21), XP055167936, Retrieved from the Internet: URL:http://www.ncbi.nlm.nih.gov/pmc/articl es/PMC2374509/pdf/82-6691112a.pdf [retrieved on 2015-02-06]
- Hongmiao Sheng ET AL: "ACCELERATED PAPER Nuclear translocation of [beta]-catenin in hereditary and carcinogen- induced intestinal adenomas", Carcinogenesis, 1 April 1998 (1998-04-01), pages 543-549, XP055167996, Retrieved from the Internet: URL:http://carcin.oxfordjournals.org/conte nt/19/4/543.full.pdf [retrieved on 2015-02-06]
- C. L. Hall ET AL: "Role of wnts in prostate cancer bone metastases", J. Cell. Biochem. vol 97(4), 2 December 2005 (2005-12-02), pages 661-672, XP055671500, Retrieved from the Internet: URL:https://onlinelibrary.wiley.com/doi/fu ll/10.1002/jcb.20735 [retrieved on 2020-02-25]

## Description

The invention relates to Wnt signaling/beta-catenin (β-catenin), and particularly, although not exclusively, to the use of compounds which can affect β-catenin translocation within the cell, for use in treating β-catenin/Wnt-signaling-related disorders. The invention extends to pharmaceutical formulations and compositions comprising such compounds.

Wnt binding to its plasma membrane receptors (e.g. Frizzled, FZDs and low density lipoprotein receptor related protein, LRPs) can activate multiple intracellular transducers including the two major transducers β-catenin (a potent transcription factor) and Ca²⁺ via the canonical or non-canonical signaling pathways, respectively. Activation of gene transcription via the canonical, Wnt/β-catenin signaling, is the most studied Wnt pathway and is known to play a key role in development and disease. The canonical signaling pathway leads to desequesterization of β-catenin from a multiprotein complex (termed stabilization) followed by activation of transcription of LEF/TCF responsive genes. However, events downstream of receptor activation, are dependent upon translocation of stabilized cytosolic β-catenin, a negatively charged 92kDa protein, traversing the nuclear envelope (NE) to enter the nucleoplasm. Activation of the non-canonical, β-catenin independent, Wnt/Ca²⁺ pathway, results in the mobilization of free intracellular calcium [Ca²⁺]ᵢ that regulates many cellular processes including cytoskeleton and cell motility of prostate cancer cells. The so-called canonical (Wnt/β-catenin signaling) and the non-canonical (Wnt/Ca²⁺ signaling) pathways are currently considered to operate independently or antagonistically.

The NE acts as a barrier that limits ion and macromolecular exchange into the nucleoplasm. NE consists of an inner nuclear membrane facing the nuclear lamina, separated by a cisterna from the outer membrane that is contiguous with the endoplasmic reticulum. Invaginations of the supramolecular nuclear pore complex (NPC) provide the only route for the exchange of matter (with an upper limit of ~90-100kDa) between the cell cyto- and nucleo-plasm. Macromolecular transport across the NE is a complex process requiring protein recognition by specific amino acid motifs (e.g. nuclear localization signal sequence (NLS) or the Armadillo domain repeat sequences) on the imported protein, first binding to the outer membrane of the NE and then traversing through the NPC by an energy expenditure mechanism such as GTPase Ran/transport receptor pathways. The 92kDa β-catenin, unlike small macromolecules (< 10kDa,) is at the upper limit for macromolecule entry through the NPC into the nucleoplasm and does not involve simple diffusion or the Ran GTPase pathway suggesting that β-catenin may encounter a considerable hurdle to traverse the NE.

The NE is also an ion-selective membrane with a dynamic trans-NE electrical potential that is responsive to changes in Ca²⁺ and K⁺ concentrations, particularly and also other ions (e.g. Na⁺). Cytosolic [Ca²⁺]ᵢ regulates nuclear Ca²⁺ concentration. An increase in nucleoplasmic Ca²⁺ facilitates transport of large macromolecules (>10kDa) across the NE, whereas a decrease in nucleoplasmic Ca²⁺ inhibits trans-NE macromolecular transport. The nucleus has been termed a 'negatively charged sink' consisting largely of negatively charged DNA and regions of nucleosome core proteins. In HeLa cells, the trans-NE potential has been measured at -43mV with an estimated electrical pore diameter of 11µm⁻². In addition to its bulk, the β-catenin protein has an overall negative charge (-20.8 at pH 7.0). Thus, the NE presents a physical as well as an electrical barrier for β-catenin entry into the nucleoplasm, indicating a physicoelectrochemical mechanism for β-catenin translocation across the NE.

Until now, precisely how β-catenin traverses the NE was not understood. β-catenin stabilization and subsequent translocation to the nucleus in response of Wnt signaling activation is a key step that is dysregulated in various β-catenin-related diseases as varied as cancer, osteoporosis and diabetes. β-catenin-related disorders are a class of diseases in which there is a dysregulation (either increased or decreased) of the translocation of β-catenin to the nucleus in response to Wnt signaling activation. When translocated to the nucleus, β-catenin acts to increase gene transcription in diseases, such as cancer and diabetes, whereas in diseases, such as osteoporosis, β-catenin translocation to the nucleus is decreased, with a subsequent decrease in gene transcription.

It is accepted knowledge that the few signaling pathways that govern major developmental processes are also causally involved in devastating diseases such as cancer. Of these few pathways, Wnt signaling represents a key molecular cascade that regulates cell fate in animals throughout their lifespan, for example, through its modulation of stem cell self-renewal. Consequently, its dysregulation, through mutations or via temporal and kinetic defects in function, are causative or associated with a variety of disorders, including cancers. It is known that Wnt signaling is upregulated in prostate cancer, and there is mounting evidence that increased Wnt-β-catenin signaling is a key step in carcinogenesis in colon, breast and other cancers. However, it has proved difficult to target downstream intracellular kinases involved in Wnt signaling. There are also multiple (>20) Wnt receptors and co-receptors and nineteen Wnt ligands that allow for significant overlap of function, but which also provide a significant challenge to the design of therapeutically effective inhibitors.

Wnt signaling, particularly TCF7 transcription factor, a nuclear transcription partner of β-catenin has also been associated with increased risk of diabetes. Some studies have suggested that the predisposing TCF7L2 variant causes a decrease in insulin secretion pancreatic β-cells. An avenue of therapy for diabetes could be targeting of pancreatic β-cell to regulate insulin release, a process that is both membrane potential and calcium dependent. High glucose enhances signaling through the cancer-associated Wnt/β-catenin pathway and may explain the increased frequency of cancer associated with obesity and diabetes.

Finally, osteoporosis, a disease that leads to increased risk of bone fracture, is an imbalance in the bone homeostasis, i.e. bone formation and bone resorption. The latter is a unique function of the osteoclast, and the former is a composite function of osteoblasts and their progenitors, osteocytes. Osteoporosis is the cause of more than 8.9 million fractures annually and is estimated to affect 200 million women worldwide - approximately one-tenth of women aged 60, one-fifth of women aged 70, two-fifths of women aged 80 and two-thirds of women aged 90 (International Osteoporosis Foundation website). Osteoporosis affects an estimated 75 million people in Europe, USA and Japan. In addition, secondary osteoporosis is caused by multiple pathologies including hyperthyroidism, diabetes and celiac disease. The bone homeostasis is regulated at multiple points in the Wnt signaling pathway. However, a key conclusion from numerous studies is that Wnt-β-catenin signaling in the osteoblast lineage inhibits osteoclastic bone resorption. Studies using transgenic mice have confirmed the critical role of Wnt signaling, particularly activation of transcription due to β-catenin translocation to the nucleus, in the regulation of bone homeostasis, i.e. activation of the Wnt-β-catenin pathway leads to increased bone mass and strength, whereas inhibition of the Wnt-β-catenin pathway leads to decreased, bone mass and strength. Because of the relationship between bone homeostasis and Wnt-β-catenin signaling, this pathway is a key target for therapeutic intervention to restore bone strength in patients at risk for fracture. Accordingly, in view of the above, it is clear that a better understanding of the underlying mechanisms of the Wnt/β-catenin and Wnt/Ca²⁺ signaling pathways would be useful for the treating a wide range of beta-catenin-related diseases, including cancer, diabetes and osteoporosis. The inventors therefore set out to investigate the relationship of these pathways in more detail.

As described in the Examples, the inventors have unexpectedly demonstrated that, contrary to current thinking, the canonical (Wnt/β-catenin signaling) and the non-canonical (Wnt/Ca²⁺ signaling) pathways do not operate independently or antagonistically. The inventors were very surprised to find that the Wnt/Ca²⁺ and Wnt/β-catenin pathways in fact act in a coordinated manner and that [Ca²⁺]ᵢ release facilitates β-catenin entry into the nucleus in mammalian cells. Indeed, the inventors have shown that six Wnt peptides (3A, 4, 5A, 7A, 9B and 10B) mobilized [Ca²⁺]ᵢ in PC₃ prostate cancer cells in a live assay using calcium dyes (with similar results in breast and bladder cells). Based upon dwell time (DT, range = 15-30s) of the calcium waveform in PC3 cells, these Wnts were classified into 3 classes: short, 3A and 5A; long, 7A and 10B; very long, 4 and 9B. Wnt-activated [Ca²⁺]ᵢ release was followed by an increase in intranuclear calcium and depolarization (a change in the transmembrane electrical potential) of both the cell and nuclear membranes, determined by using FM4-64 dye. In cells treated with Wnts 5A, 9B and 10B, paradigm substrates for each Wnt class, increased [Ca²⁺]ᵢ was followed by β-catenin translocation into the nucleus in PC3, MCF7 and 253J, prostate, breast and bladder cancer cell lines. Surprisingly, both the increase in Wnt 5A, 9B and 10B induced [Ca²⁺]ᵢ release and β-catenin translocation are suppressed by thapsigargin in PC3 cell line.

In eukaryotes, cell membrane potential is mainly a function of distribution of Na⁺, K⁺ and Ca²⁺ across the cell membrane. ATPase is also known to influence the electrical potential on a cell membrane. Release of [Ca²⁺]ᵢ from stores is also tightly coordinated with membrane potential changes via ion channels and may itself be regulated by the prevailing membrane potential. The inventors used techniques of [Ca²⁺]ᵢ release and immunocytochemistry in cell lines to test this hypothesis. They have demonstrated that modulation of the cell membrane potential, or blockade of ion channels, inhibits Wnt-mediated [Ca²⁺]ᵢ release and translocation of β-catenin to the nucleus, and thereby decreases cell proliferation.

In view of these unexpected findings, the inventors have proposed the first ever convergent model of Wnt signaling network where Ca²⁺ and β-catenin pathways can act in a coordinated, interdependent, rather than independent, manner, as previously thought. Based on this novel convergent model, the inventors believe that a range of beta-catenin-related diseases, including cancer, diabetes and osteoporosis, can now be effectively treated using compounds that regulate the electrical potential of the cell membrane. As such, these results present an exciting new therapeutic avenue for treating β-catenin related diseases.

Hence, in a first aspect, there is provided a cell membrane electrical potential-regulating agent comprising Dofetilide, which modulates β-catenin translocation in the cell, for use in treating a β-catenin-related disease.

In a second aspect, there is provided a method of treating, preventing or ameliorating a β-catenin-related disease, the method comprising administering, to a subject in need of such treatment, a therapeutically effective amount of a cell membrane electrical potential-regulating agent comprising Dofetilide, which modulates β-catenin translocation in the cell.

The inventors have unexpectedly discovered that Wnt signaling-induced nuclear translocation of β-catenin can be controlled by targeting the electrical activity of the cell membrane, and thereby altering that of the nuclear membrane, for example by modulating trans-cellular potassium gradient via channel or transporter activity or the availability of free intracellular calcium ([Ca²⁺]ᵢ). As such, small molecule chemical blockers that alter ion transport across the cell membrane already in therapeutic use for other indications can be used to regulate β-catenin translocation to the nucleus where it is known to drive proliferative diseases, such as cancer. Accordingly, using ion channel or transporter blocking compounds, β-catenin translocation to the nucleus can be restricted resulting in a decrease in cell proliferation, and thereby used to treat cancer. Conversely, use of compounds which activate certain ion channels results in the increase of β-catenin translocation to the nucleus, and can be harnessed to treat osteoporosis.

β-catenin and ([Ca²⁺]ᵢ) release are key transducers of Wnt signaling. In Example 1, the inventors have provided a new convergent model of Wnt signaling in which [Ca²⁺]ᵢ release facilitates β-catenin entry into the nucleus. β-catenin is a potent transcription factor and its translocation to the nucleus is a key step in many diseases including cancer and diabetes, where there is increased translocation of β-catenin and gene transcription, or osteoporosis where there is too little β-catenin translocation and gene transcription. There are over 20 receptors and co-receptors for up to 19 Wnt ligands that render the conventional pharmacological approaches of designing receptor antagonists a difficult strategy. Downstream targets such as GSK₃β kinase, a key enzyme and part of the destruction complex, that regulates availability of the stabilized cytosolic β-catenin, is a cytosolic enzyme and presents all too often encountered problems of drug entry and accessibility. The inventors have however shown in the Examples that multiple Wnt ligands activate intracellular calcium stores [Ca²⁺]i and also increase β-catenin translocation to the nucleus where it acts as a transcription factor. They have also shown that increase in [Ca²⁺]ᵢ results in depolarization of cell and nuclear membrane potentials that helps facilitate entry of β-catenin, stabilized through activation of Wnt signaling, into the nucleus. The inventors further demonstrated that inhibition or depletion of intracellular calcium stores by thapsigargin (a Ca²⁺ ATPase inhibitor) abolishes Wnt mediated [Ca²⁺]ᵢ release and β-catenin translocation to the nucleus. It is clear that both Ca²⁺ and membrane potential play a central role in entry of β-catenin into the nucleus, a novel observation that led them to formulate a completely new model for Wnt signaling.

Advantageously, targeting the β-catenin translocation to the nucleus by modulating cellular processes or cell membrane proteins, not directly or obviously related to Wnt signaling pathway, is an attractive proposition. The inventors investigated if regulating membrane potential could modulate β-catenin entry into the nucleus, and the results clearly demonstrate that it is possible to modulate β-catenin translocation through regulation of cell and nuclear membrane potential via the blockade of ion transporters or channels (e.g. Na⁺, K⁺ or Ca²⁺) or direct inhibition of Wnt ligand binding to its receptors by agents that are known to block channels (e.g. by tolbutamide and CsCl). Hence, these results present an exciting new therapeutic avenue for treating β-catenin related diseases.

When translocated to the nucleus, β-catenin increases gene transcription in cancer and diabetes. Accordingly, in one preferred embodiment of the invention, the cell membrane electrical potential-regulating agent is arranged to decrease beta-catenin translocation to the cell's nucleus, and is used to treat cancer or diabetes. The agent is preferably used to treat Type 2 diabetes. For example, cancers that may be treated in this way may include cancer of the colon, prostate, breast, pituitary gland, pancreas, oesophagus, penis or liver. The cancer may also be diabetes-induced cancer. Preferably, the cancer may be Barrett Oesophagus, which is a peculiar form of healing in the lining of the distal oesophagus that occurs in response to chronic gastro-oesophageal reflux, predisposing the patient to neoplastic growth.

The cell membrane electrical potential-regulating agent comprises Dofetilide.

Advantageously, many of these compounds are already being used in therapy and present an easy route to therapy for treatment of cancer or diabetes. Table 1 describes the putative dosages, all within current clinical guidelines, for the compounds of interest.

2The agents according to the invention may be combined in compositions having a number of different forms depending, in particular, on the manner in which the composition is to be used. Thus, for example, the composition may be in the form of a powder, tablet, capsule, liquid, ointment, cream, gel, hydrogel, aerosol, spray, micellar solution, transdermal patch, liposome suspension or any other suitable form that may be administered to a person or animal in need of treatment. It will be appreciated that the vehicle of medicaments according to the invention should be one which is well-tolerated by the subject to whom it is given.

Medicaments comprising agents according to the invention may be used in a number of ways. For instance, oral administration may be required, in which case the agents may be contained within a composition that may, for example, be ingested orally in the form of a tablet, capsule or liquid. Compositions comprising agents of the invention may be administered by inhalation (e.g. intranasally). Compositions may also be formulated for topical use. For instance, creams or ointments may be applied to the skin.

Agents according to the invention may also be incorporated within a slow- or delayed-release device. Such devices may, for example, be inserted on or under the skin, and the medicament may be released over weeks or even months. The device may be located at least adjacent the treatment site. Such devices may be particularly advantageous when long-term treatment with agents used according to the invention is required and which would normally require frequent administration (e.g. at least daily injection).

In a preferred embodiment, agents and compositions according to the invention may be administered to a subject by injection into the blood stream or directly into a site requiring treatment. Injections may be intravenous (bolus or infusion) or subcutaneous (bolus or infusion), or intradermal (bolus or infusion).

It will be appreciated that the amount of the agent that is required is determined by its biological activity and bioavailability, which in turn depends on the mode of administration, the physiochemical properties of the agent (for example an antibody), and whether it is being used as a monotherapy, or in a combined therapy. The frequency of administration will also be influenced by the half-life of the agent within the subject being treated. Optimal dosages to be administered may be determined by those skilled in the art, and will vary with the particular agent in use, the strength of the pharmaceutical composition, the mode of administration, and the advancement of the β-catenin-related disease, such as cancer, diabetes or osteoporosis. Additional factors depending on the particular subject being treated will result in a need to adjust dosages, including subject age, weight, gender, diet, and time of administration.

Exemplary doses of compounds used in accordance with the invention are provided in Table 1. Generally, a daily dose of between 0.01µg/kg of body weight and 500mg/kg of body weight of the agent according to the invention may be used for treating, ameliorating, or preventing prostate cancer More preferably, the daily dose is between 0.01mg/kg of body weight and 400mg/kg of body weight, more preferably between 0.1mg/kg and 200mg/kg body weight, and most preferably between approximately 1mg/kg and 100mg/kg body weight.

The agent may be administered before, during or after onset of the disease to be treated. Daily doses may be given as a single administration (e.g. a single daily injection). Alternatively, the agent may require administration twice or more times during a day. As an example, agents may be administered as two (or more depending upon the severity of the β-catenin-related disease being treated) daily doses of between 25mg and 7000 mg (i.e. assuming a body weight of 70 kg). A patient receiving treatment may take a first dose upon waking and then a second dose in the evening (if on a two dose regime) or at 3- or 4-hourly intervals thereafter. Alternatively, a slow release device may be used to provide optimal doses of agents according to the invention to a patient without the need to administer repeated doses.

Known procedures, such as those conventionally employed by the pharmaceutical industry (e.g. *in vivo* experimentation, clinical trials, etc.), may be used to form specific formulations comprising the agents according to the invention and precise therapeutic regimes (such as daily doses of the agents and the frequency of administration). The inventors believe that they are the first to describe a pharmaceutical composition for treating a β-catenin-related disease, such as cancer, diabetes or osteoporosis, based on the use of the agents of the invention, which are designed to regulate the electrical potential of the cell membrane and thereby influence intracellular calcium ion availability, or a combination of calcium, potassium or sodium ions that can influence cell and nuclear membrane potential and modulate (i.e. increase or decrease) the amount of β-catenin translocation to the nucleus.

Hence, in a third aspect of the invention, there is provided a β-catenin-related disease treatment composition agent comprising Dofetilide, which modulates β-catenin translocation in the cell, and a pharmaceutically acceptable vehicle.

The pharmaceutical composition can be used in the therapeutic amelioration, prevention or treatment of any β-catenin-related disease in a subject caused by either too much or too little β-catenin translocation to the nucleus.

The invention also provides in a fourth aspect, a process for making the composition according to the third aspect, the process comprising contacting a therapeutically effective amount of a cell membrane electrical potential-regulating agent, which modulates β-catenin translocation in the cell, and a pharmaceutically acceptable vehicle.

A "subject" may be a vertebrate, mammal, or domestic animal. Hence, agents, compositions and medicaments according to the invention may be used to treat any mammal, for example livestock (e.g. a horse), pets, or may be used in other veterinary applications. Most preferably, however, the subject is a human being.

A "therapeutically effective amount" of agent is any amount which, when administered to a subject, is the amount of drug that is needed to treat the target disease, or produce the desired effect, i.e. modulate the electrical potential of the cell membrane in order to modulate the amount of β-catenin translocation to the nucleus.

For example, the therapeutically effective amount of agent used may be from about 0.01 mg to about 800 mg, and preferably from about 0.01 mg to about 500 mg. It is preferred that the amount of agent is an amount from about 0.1 mg to about 250 mg, and most preferably from about 0.1 mg to about 20 mg.

A "pharmaceutically acceptable vehicle" as referred to herein, is any known compound or combination of known compounds that are known to those skilled in the art to be useful in formulating pharmaceutical compositions.

In one embodiment, the pharmaceutically acceptable vehicle may be a solid, and the composition may be in the form of a powder or tablet. A solid pharmaceutically acceptable vehicle may include one or more substances which may also act as flavouring agents, lubricants, solubilisers, suspending agents, dyes, fillers, glidants, compression aids, inert binders, sweeteners, preservatives, dyes, coatings, or tablet-disintegrating agents. The vehicle may also be an encapsulating material. In powders, the vehicle is a finely divided solid that is in admixture with the finely divided active agents according to the invention. In tablets, the active agent may be mixed with a vehicle having the necessary compression properties in suitable proportions and compacted in the shape and size desired. The powders and tablets preferably contain up to 99% of the active agents. Suitable solid vehicles include, for example calcium phosphate, magnesium stearate, talc, sugars, lactose, dextrin, starch, gelatin, cellulose, polyvinylpyrrolidine, low melting waxes and ion exchange resins. In another embodiment, the pharmaceutical vehicle may be a gel and the composition may be in the form of a cream or the like.

However, the pharmaceutical vehicle may be a liquid, and the pharmaceutical composition is in the form of a solution. Liquid vehicles are used in preparing solutions, suspensions, emulsions, syrups, elixirs and pressurized compositions. The active agent according to the invention may be dissolved or suspended in a pharmaceutically acceptable liquid vehicle such as water, an organic solvent, a mixture of both or pharmaceutically acceptable oils or fats. The liquid vehicle can contain other suitable pharmaceutical additives such as solubilisers, emulsifiers, buffers, preservatives, sweeteners, flavouring agents, suspending agents, thickening agents, colours, viscosity regulators, stabilizers or osmoregulators. Suitable examples of liquid vehicles for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the vehicle can also be an oily ester such as ethyl oleate and isopropyl myristate. Sterile liquid vehicles are useful in sterile liquid form compositions for parenteral administration. The liquid vehicle for pressurized compositions can be a halogenated hydrocarbon or other pharmaceutically acceptable propellant.

Liquid pharmaceutical compositions, which are sterile solutions or suspensions, can be utilized by, for example, intramuscular, intrathecal, epidural, intraperitoneal, intravenous and particularly subcutaneous injection. The agent may be prepared as a sterile solid composition that may be dissolved or suspended at the time of administration using sterile water, saline, or other appropriate sterile injectable medium.

The agents and compositions of the invention may be administered orally in the form of a sterile solution or suspension containing other solutes or suspending agents (for example, enough saline or glucose to make the solution isotonic), bile salts, acacia, gelatin, sorbitan monoleate, polysorbate 80 (oleate esters of sorbitol and its anhydrides copolymerized with ethylene oxide) and the like. The agents used according to the invention can also be administered orally either in liquid or solid composition form. Compositions suitable for oral administration include solid forms, such as pills, capsules, granules, tablets, and powders, and liquid forms, such as solutions, syrups, elixirs, and suspensions. Forms useful for parenteral administration include sterile solutions, emulsions, and suspensions.

All of the features described herein (including any accompanying claims, abstract and drawings), and/or all of the steps of any method or process so disclosed, may be combined with any of the above aspects in any combination, except combinations where at least some of such features and/or steps are mutually exclusive.

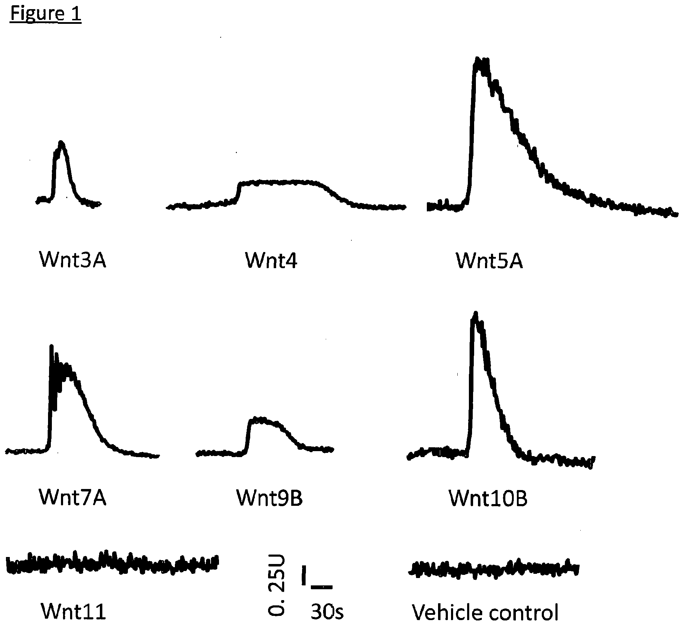

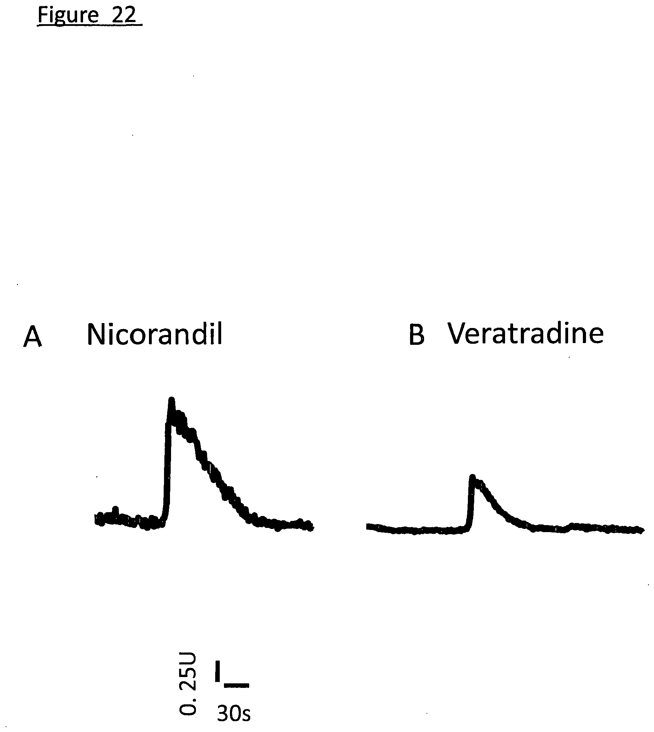

For a better understanding of the invention, and to show how embodiments of the same may be carried into effect, reference will now be made, by way of example, to the accompanying Figures, in which:-
Figure 1 is a representative trace of intracellular calcium release in PC₃ cells in response to addition of 100ng/ml Wnt ligand peptide (*Wnt3A, grey; Wnt4, red; Wnt5A, green; Wnt7A, pink; Wnt9B, blue; Wnt10B, brown; Wnt11, violet; vehicle control, yellow*). All Wnts tested, except Wnt11, induce intracellular calcium mobilization in PC3 prostate cancer cell line. X-axis is time (*s*) and Y-axis is ratio of Fluo4/Fura Red. The waveform for each ligand was used to calculate time constants (rise, dwell and fall time) of calcium release;

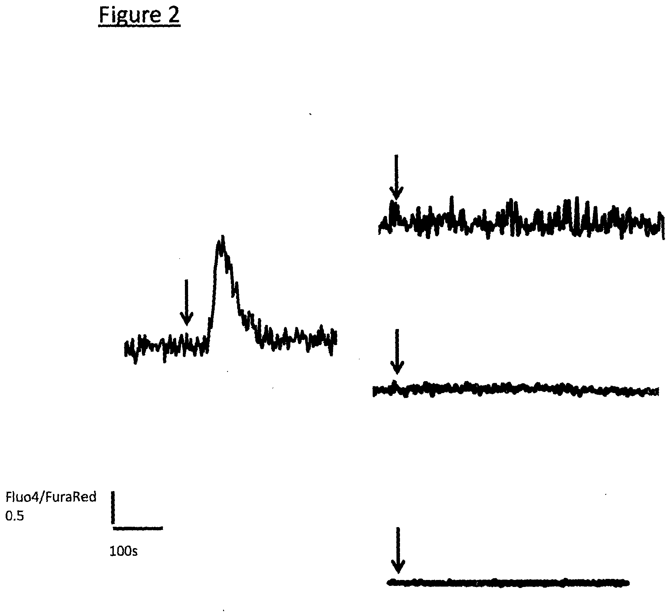

Figure 2 reflects [Ca²⁺]ᵢ measurements made as described in experimental procedures using Fluo4 and FuraRed dyes. Thapsigargin (at 5µM final concentration) was added at the time indicated (arrow). A representative trace of a calcium transient (cyan) evoked after the addition of thapsigargin is shown. The calcium signal (ratio of Fluo4/FuraRed) declined after an initial rise to reach the baseline within 120s. In PC3 cells incubated with thapsigargin (5µM) for 15min, addition of Wnt5A (100ng/ml, green), Wnt9B (200ng/ml, blue) or Wnt 10B (200ng/ml, brown) did not evoke an increase in [Ca²⁺]i as seen in untreated PC3 cells (Figure 1);

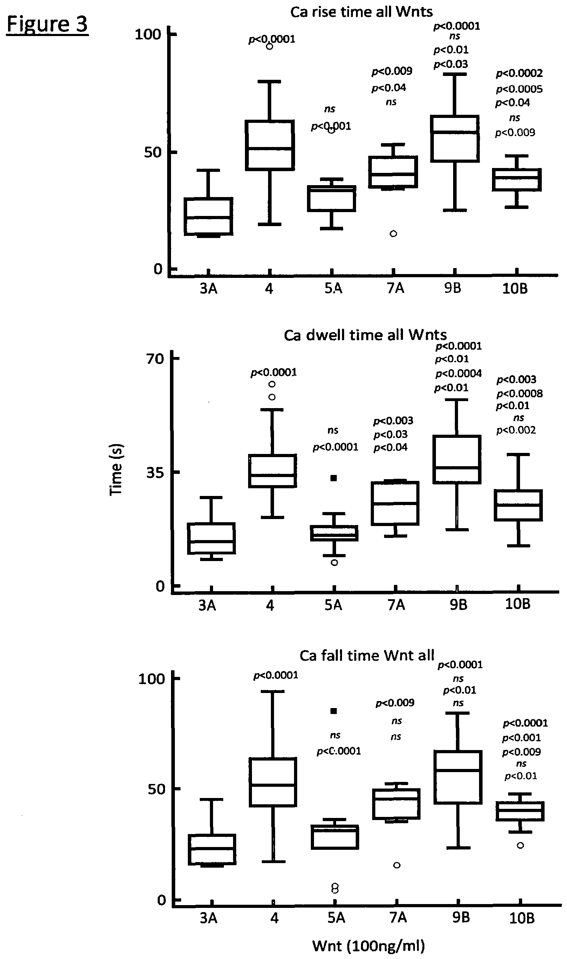

Figure 3 is a box-plot for time constants of the Wnt induced calcium waveform: The time constants (A, rise, B, dwell and C, fall times) were calculated from calcium waveform generated by the addition of each Wnt ligand (100ng/ml) to PC3 cells. Significance of difference was calculated using Mann-Whitney U test. All time constants were compared to Wnt3A, primarily; subsequent comparisons (e.g. Wnt4 to Wnt5A, Wnt4 to Wnt7A etc) are listed in descending order on top of box plots for each subsequent Wnt. *n.s.* = difference was not statistically significant;

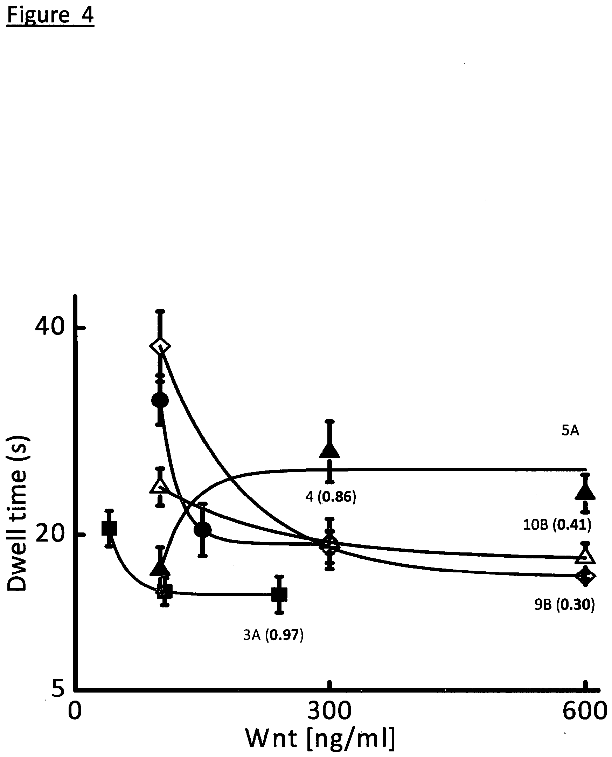

Figure 4 shows Wnts that activate [Ca²⁺]ᵢ release show negative co-operativity. Wnts 3A (*grey*), 4 (*red*), 9B (*blue*) and 10B (*dark blue*) showed decreasing and significant difference between at least two incremental increasing concentrations for the dwell time for [Ca²⁺]ᵢ release. The dwell time constant at increasing Wnt concentration are fitted to a first order reaction equation (yo+A1e (-x/t1)), where yo=Y offset, A1=amplitude and t1=decay constant. The rate constant for the reaction (*k*) is given in brackets for each Wnt ligand. No significant difference was found in the dwell times for different concentration for Wnt7A and therefore the data is not considered for this analysis. Wnt5A (*green*) showed a classic Michaelis-Menten kinetics with an estimated K_{1/2} of 67ng/ml;

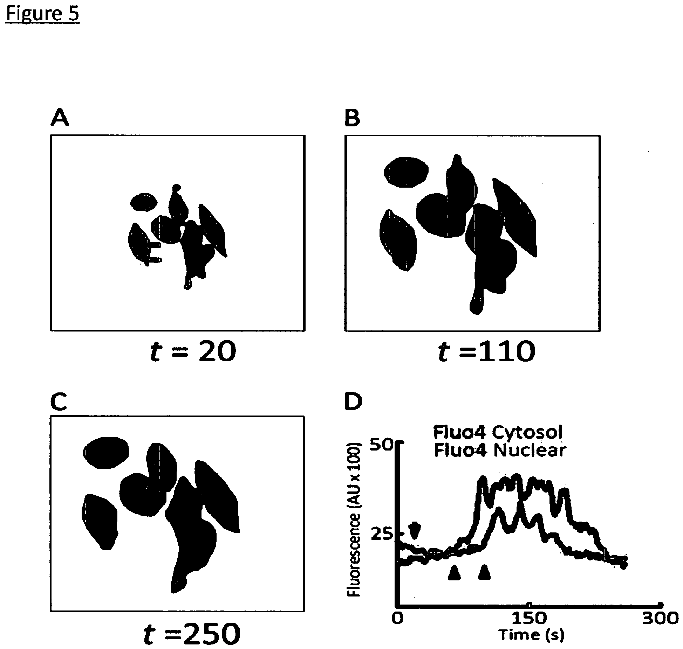

Figure 5 shows [Ca²⁺]ᵢ release results in an increase in intranuclear Ca²⁺ concentration. Snapshots of three time points (A-C) at t=20, 110 and 250s (A-C) in PC3 cells loaded with Fluo4 (green) and FM4 (red) are shown and treated with Wnt5A (100ng/ml). Quantitative expression of the increase in cytosolic ([Ca²⁺]ᵢ , red line) and intranuclear Ca²⁺ (violet line) is given in (D). (A) At t=20, steady state, baseline [Ca²⁺]ᵢ as a function of Fluo4 intensity is shown that shows an increase at (B) t=110s, subsequent to the addition of Wnt5A (black arrow), increase in [Ca²⁺]ᵢ is initiated (red arrow) with an increase in intranuclear Ca²⁺ following shortly (~33s) later (violet arrow). At t=250s (C) there is an increase in the FM4 signal in the cell and nuclear membranes (brown arrows). A representative from three independent measurements is shown. Imaging was performed on Leica SP2 confocal and DMRB microscope with 20× 0.8NA water dipping objective and 12bit grey depth using similar conditions to the FV1000;

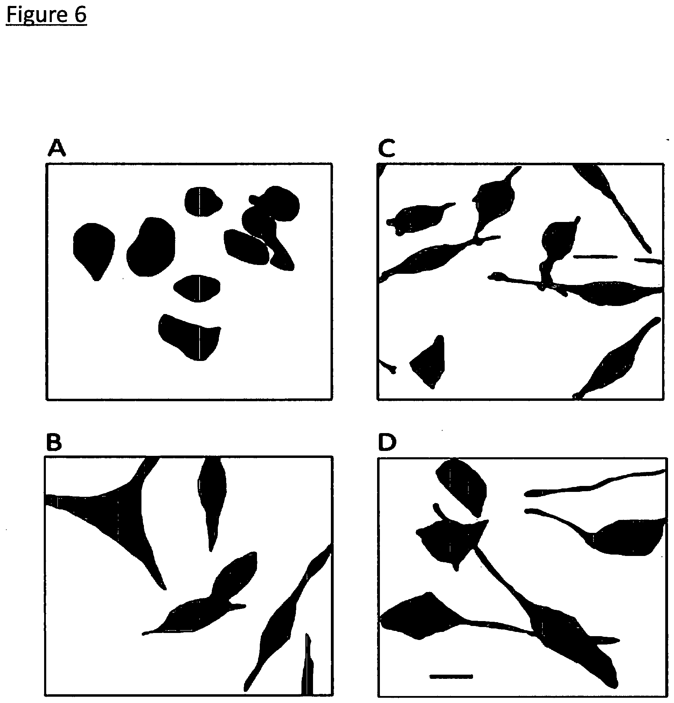

Figure 6 shows β-catenin (red) translocation to the nucleus (counterstained with DAPI, blue) in response to activation by Wnt ligands (100ng/ml) in in PC3 prostate cancer cell line control (A) or after activation with Wn5A (B), Wnt 9B (C) and Wnt 10B (D). Cells were grown in 8 well chamber slides, treated with Wnt ligands, fixed and stained for β-catenin using protocols described in materials and methods. Z-stacks obtained using an Olympus confocal system are shown. Representative images of 3 individual experiments are shown. Scale bar =10µm;

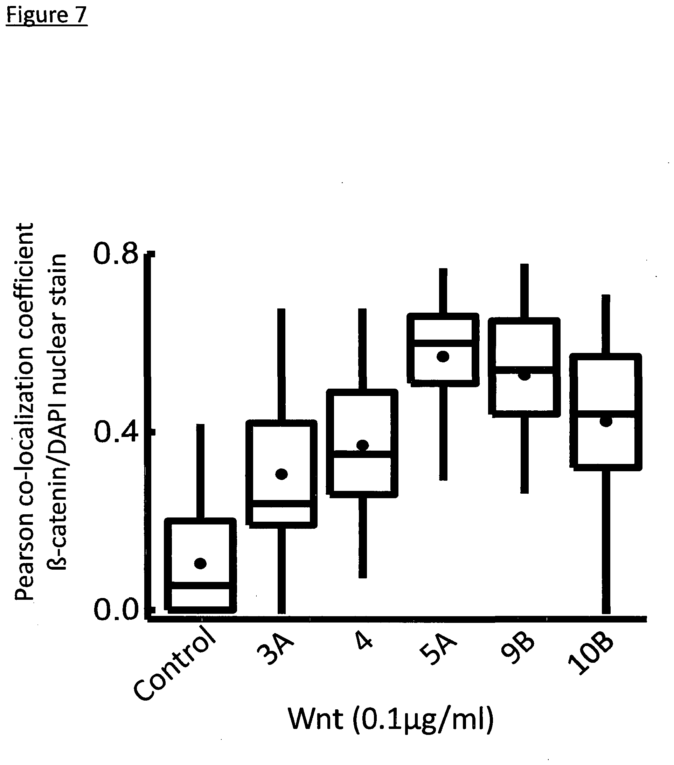

Figure 7 shows box plots of calculated Pearson co-localization coefficient of β-catenin translocation to the nucleus. Immunohistochemistry and image analysis was performed as described in experimental procedures. Between 20-97 cells from 2-3 independent experiments were analyzed. Circles represent means of Pearson colocalization coefficient for each condition. Cy3 (label for β-catenin) showed significantly (p < 0.001, Mann Whitney test) increased co-localization coefficients for Wnt (100ng/ml) treated vs control cells;

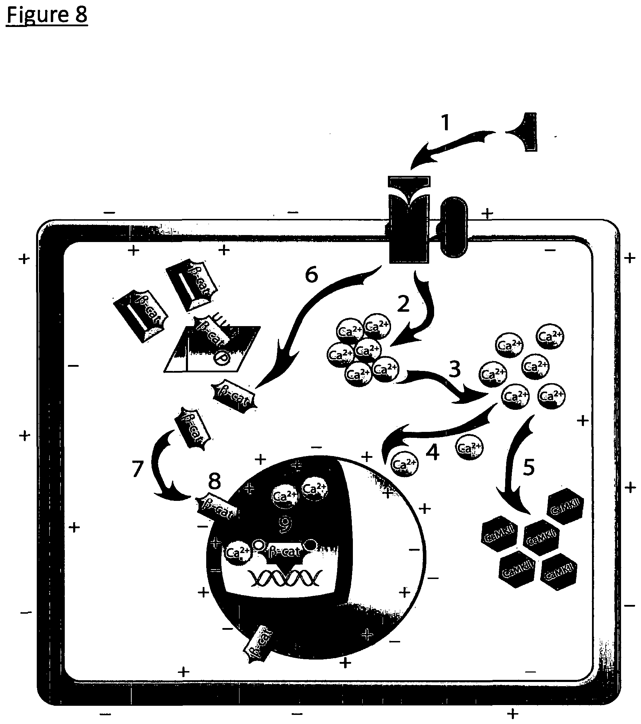

Figure 8 shows a convergent model for the canonical (Wnt/β-catenin) and non-canonical (Wnt/Ca²⁺) Wnt signaling, in mammalian cell lines (only the cell membrane and the nucleus are shown in the cartoon for clarity);

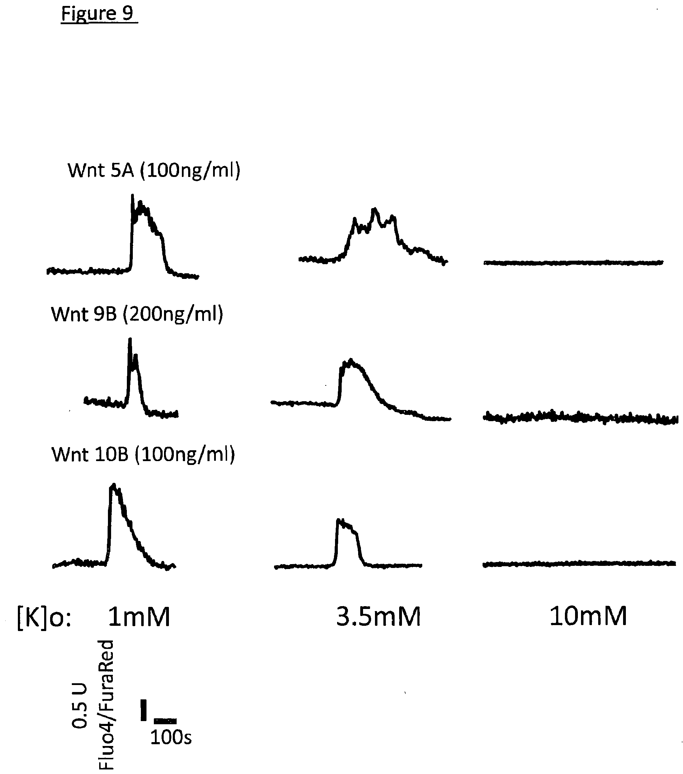

Figure 9 is a representative trace of intracellular calcium release in PC3 cells in response to addition of Wnt ligand peptide *Wnt5A, green; Wnt9B, blue; Wnt10B, brown* in the presence of varying external potassium [K⁺]o. The figure shows that trans-cellular K⁺ concentration gradient regulates the activation of intracellular Ca²⁺ in response to Wnt signaling;

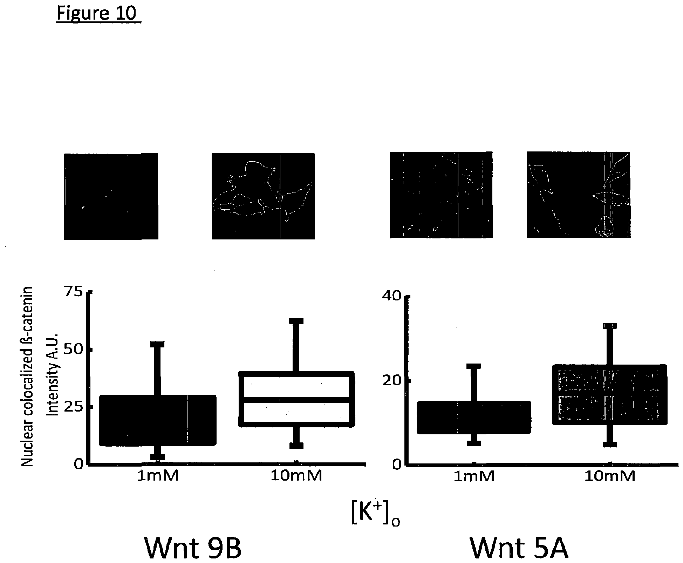

Figure 10 - Top: Representative confocal micrograph of β-catenin staining (red) in PC3 cells in the presence of varying external K⁺ when Wnt signaling is activated by Wnt 9B; nucleus is counterstained with DAPI (blue). Bottom: quantitation of β-catenin translocated to the nucleus. Increasing external K⁺ and thereby depolarizing the cell membrane potential increases the translocation of β-catenin in response to Wnt9B. The Figure shows that External K⁺ and β-catenin translocation to the nucleus in PC3 cells subsequent to activation of Wnt signaling;

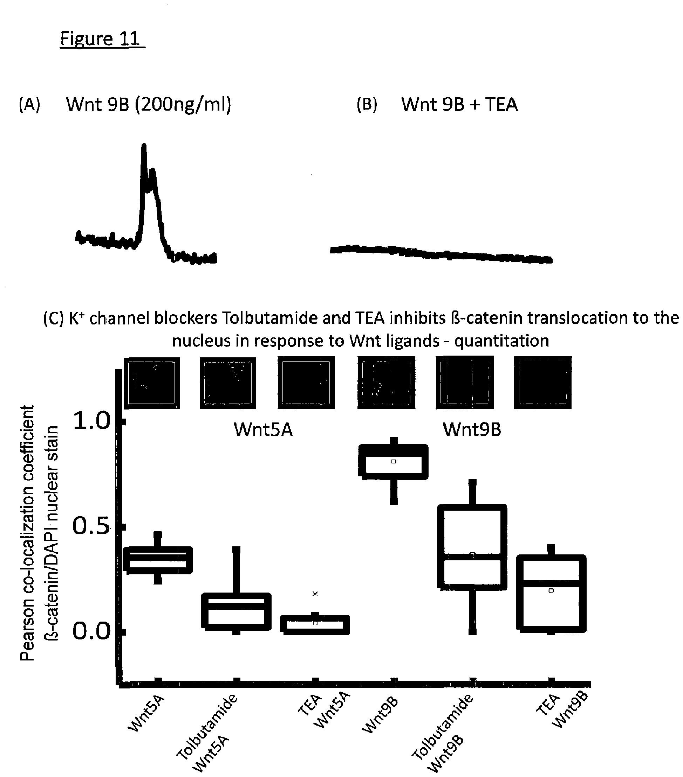

Figure 11 shows that Wnt mediated intracellular calcium release (A) is abolished by treatment of cells with membrane potential regulators (B, e.g. tetraethylammonium, TEA, a K⁺ channel inhibitor). (C) Inhibitors of K⁺ channels (1-15mM) also reduce Wnt mediated translocation of beta catenin to nucleus. Representative confocal micrograph of β-catenin staining (green) in PC3 cells treated with K⁺ channel inhibitors and Wnt5A (red bordered) and 9B (blue bordered) are given above the corresponding box plots. The Figure shows that regulators of membrane potential modulate Wnt mediated intracellular calcium release and β-catenin translocation;

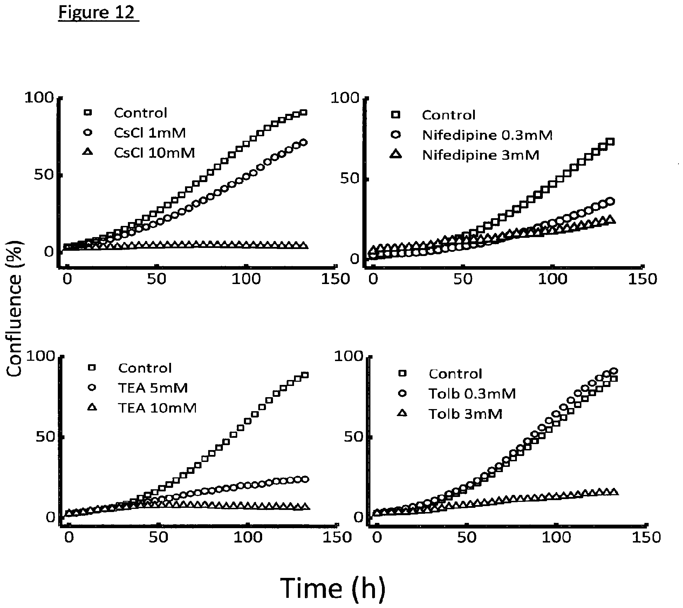

Figure 12 shows that regulators of cell membrane potential (K⁺ and Ca²⁺ channel inhibitors) reduced cell proliferation rate of PC3 cells. X-axis is (h) and y-axis is confluence of cell growth measured using live imaging. The Figure shows that CsCl, Nifedipine, TEA and Tolbutamide inhibit proliferation of PC3 cells;

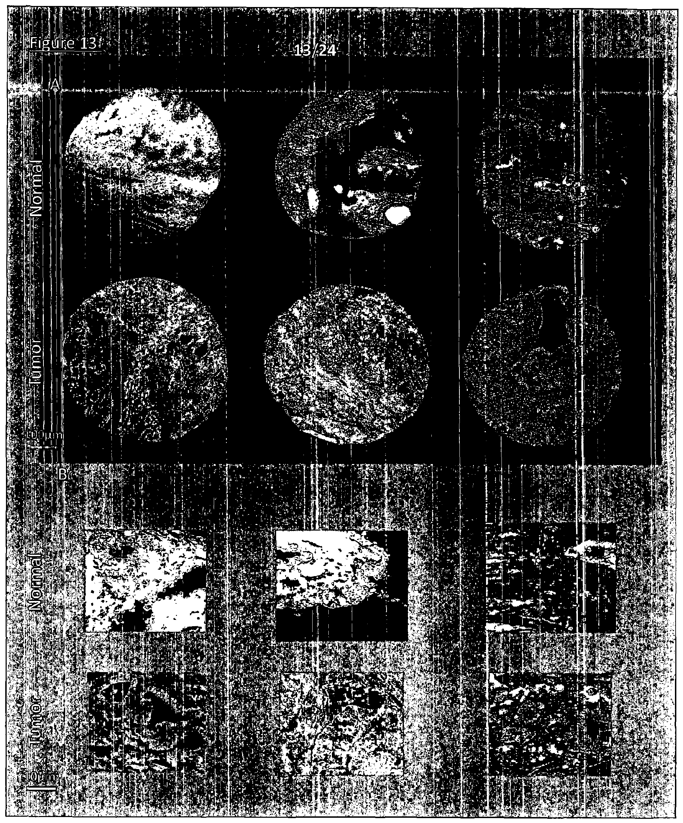

Figure 13 shows that Wnt signaling proteins are upregulated in prostate cancer tissue. Representative multilabel images for prostate tissue stained with four Wnt signaling antibodies (A): normal (tumor adjacent, top) and tumor (bottom) samples from 3 patients are shown. Composite, overlay, of four fluorophores (FITC, Alexa Fluor-405, Cy3 and Cy5 pseudo colored for MMP7 (green), β-catenin (cyan), Cyclin D1 (red) and c-MYC (blue), respectively) is shown. There is a visible and quantitative difference (not shown) in the expression of Wnt signaling targets in normal compared to cancerous prostate samples. The tissue samples are from a centre that is part of the collaborative. (B) is a high magnification image of cores shown in (A);

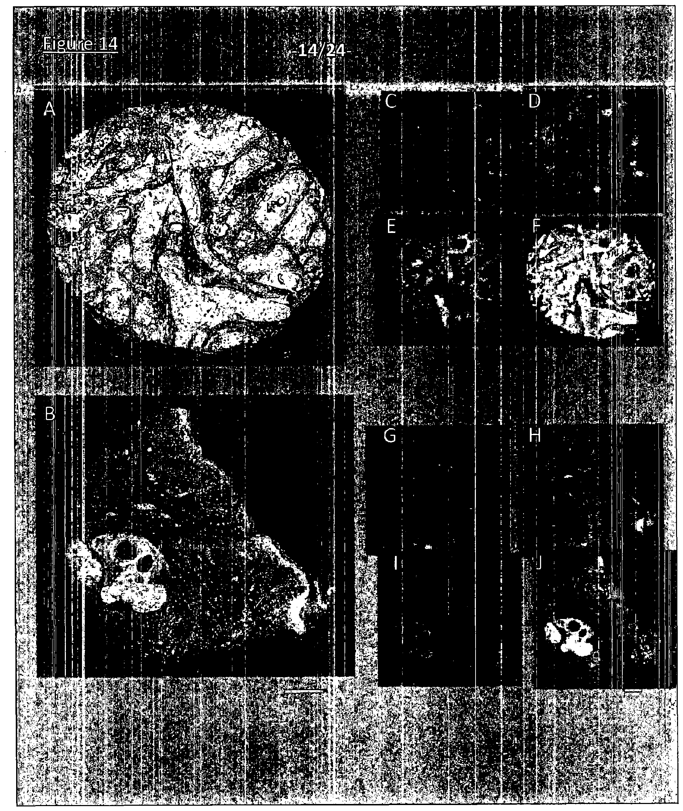

Figure 14 shows that Wnt signaling proteins are up-regulated in human penile squamous cell carcinoma: Representative images from Zeiss AxioScan Zi slide scanner. Composite, overlay, of four fluorophores (FITC, Alexa Fluor-405, Cy3 and Cy5 for MMP7, Wnt4, Cyclin D1 and c-MYC, respectively) in (A) PeCa tissue cores (core B2 of PE241) and (B) control (core D5 of PE241, see methods). C-F are individual images used to construct the composite of PeCa tissue core, for MMP7 (C, green), Wnt4 (D, blue), Cyclin D1 (E, orange) and c-MYC (F, red). G-J are individual images used to construct the composite of control tissue core, for MMP7 (G, green), Wnt4 (H, blue), Cyclin D1 (I, orange) and c-MYC (J, red). Quantitative analysis was performed on the non-enhanced, original, gray images for which all settings were identical in all tissue core images analyzed. Scale bar= 250 µm;

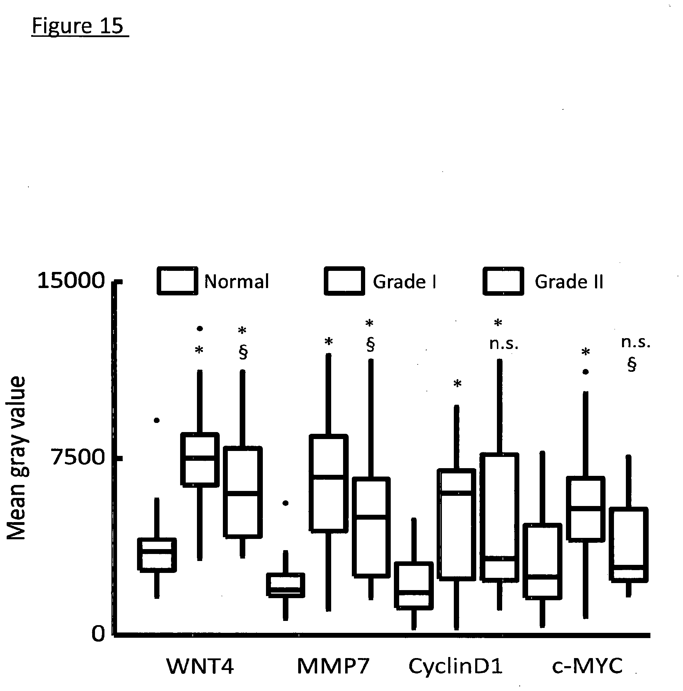

Figure 15 is a box plot of the quantitation of expression of WNT4, MMP7, CD1 and c-MYC in PeCa tissue classified according to pathological grade. 97% of the samples used in this study were classified as grade I or II. The data presented in Table 1 was segregated into expression according to pathological grades and plotted as box plots. Filled circles are 99% value and horizontal line within the box is the median value. Protein expression (mean gray value per core) in control samples (green, n=37); malignant tumor sample classified as grade I by a pathologist (blue, n=82) or grade II (purple, n=18). All proteins showed a significant difference in expression between control and malignant tumor samples. Significance of difference was measured using Mann-Whitney U test: ^{∗}=p<0.001 and ^{§}=p<0.01 between control and malignant grade I or grade II samples or within grade I and grade II. There was no significant difference in protein expression for any marker between grade I and grade II samples;

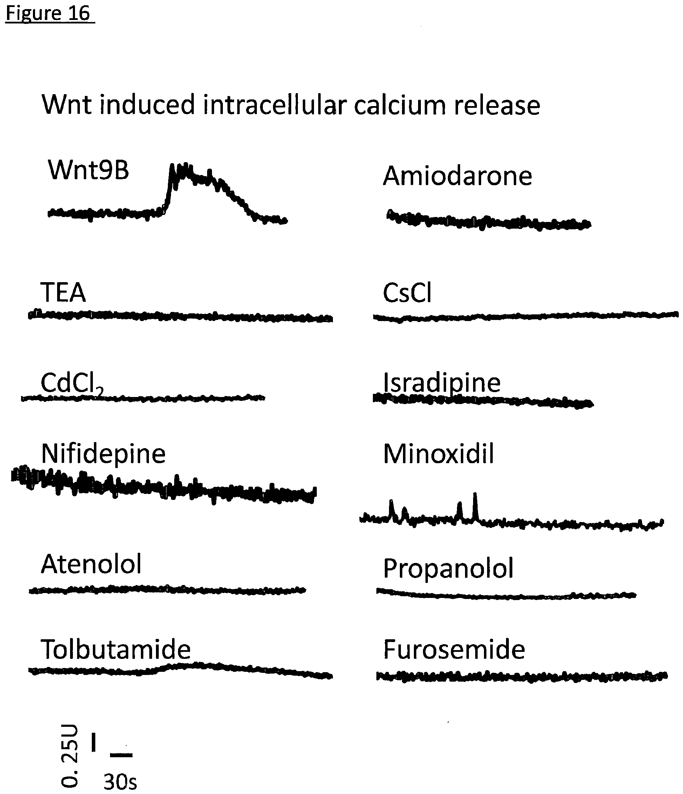

Figure 16 shows that membrane Potential Regulating Compounds (MPRCs) inhibit Wnt signaling as measured by Wnt induced calcium release (Thrasivoulou et al, 2013, J Biol Chem J Biol Chem 288: 35651-35659). Representative trace of Wnt induced calcium release (A, Wnt 9B at 0.1µg/ml is shown as an example). The Wnt ligand induced calcium release was inhibited after preincubation for 5 min with MPRCs (CsCl, 5mM; CdCl2 imM (Ca-channel inhibitor); Tetraethyl ammonium (TEA), 10mM; Amiodarone, 75µM; Tolbutamide, imM; Isradipine 100µM; Nifedipine, imM; Minoxidil, 25µM; Atenolol, 37.5µM; Propanolol, 50µM; Furosemide, 150µM). X-axis is time (s) and Y-axis is ratio of Fluo4/Fura Red. Detailed methods are described in Thrasivoulou et al, 2013, J Biol Chem J Biol Chem 288: 35651-35659;

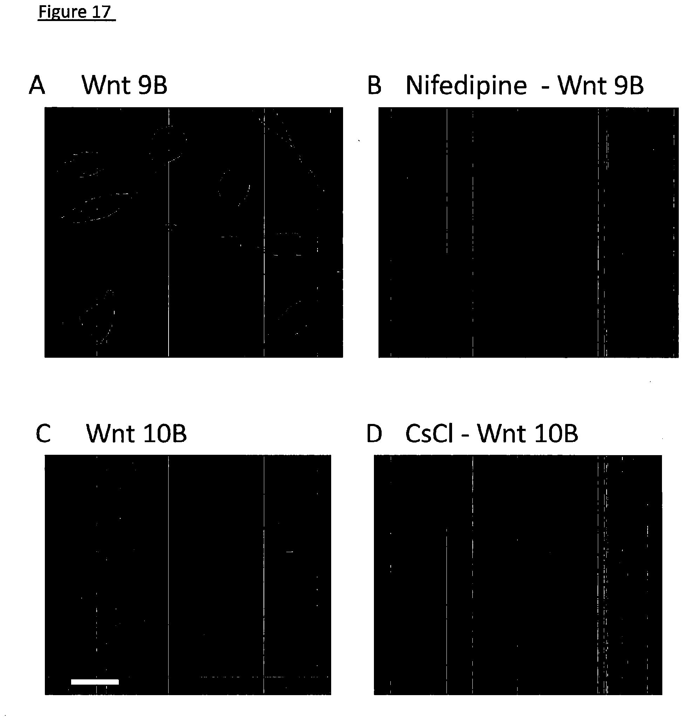

Figure 17 shows that membrane Potential Regulating Compounds (MPRCs) inhibit Wnt signaling as measured by Wnt mediated β-catenin translocation (Thrasivoulou et al, 2013, J Biol Chem J Biol Chem 288: 35651-35659) in PC3 prostate cancer cell line. Cells were treated with Wnts (shown here as an example) with or without preincubation (5 min) with MPRCs at 37°C in a CO₂ incubator. Wnts were added and cells were incubated for a further 20-30min, washed, fixed and stained according to the protocols described in Thrasivoulou et al, 2013, J Biol Chem J Biol Chem 288: 35651-35659. β-catenin is red; cell nucleus is blue and was counter-stained with 4',6-diamidino-2-phenylindole (DAPI). Data for other MPRCs is given in Figure 11;

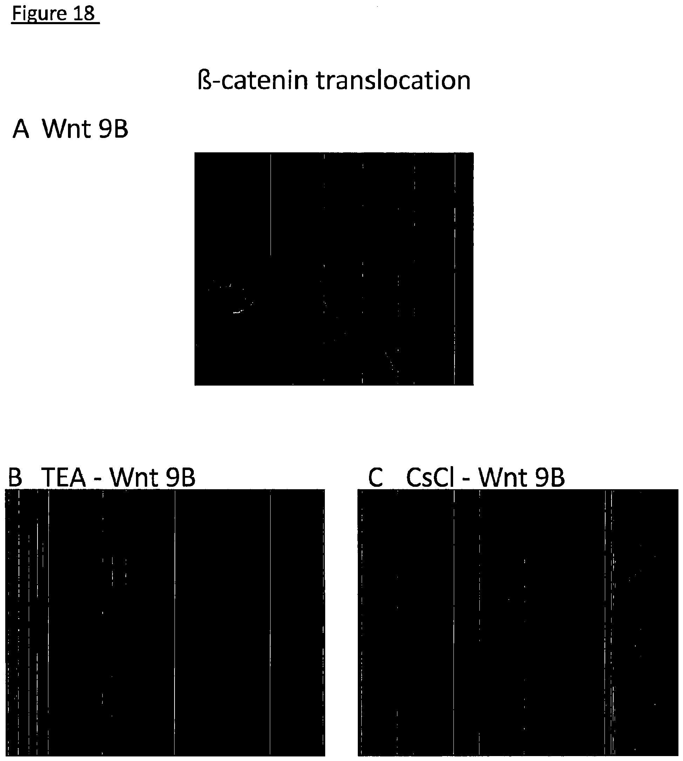

Figure 18 shows that Membrane Potential Regulating Compounds (MPRCs) inhibit Wnt signaling as measured by Wnt mediated β-catenin translocation (Thrasivoulou et al, 2013, J Biol Chem J Biol Chem 288: 35651-35659) in MCF 7 breast cancer cell line. β-catenin is red; cell nucleus is blue and was counter-stained with 4',6-diamidino-2-phenylindole (DAPI);

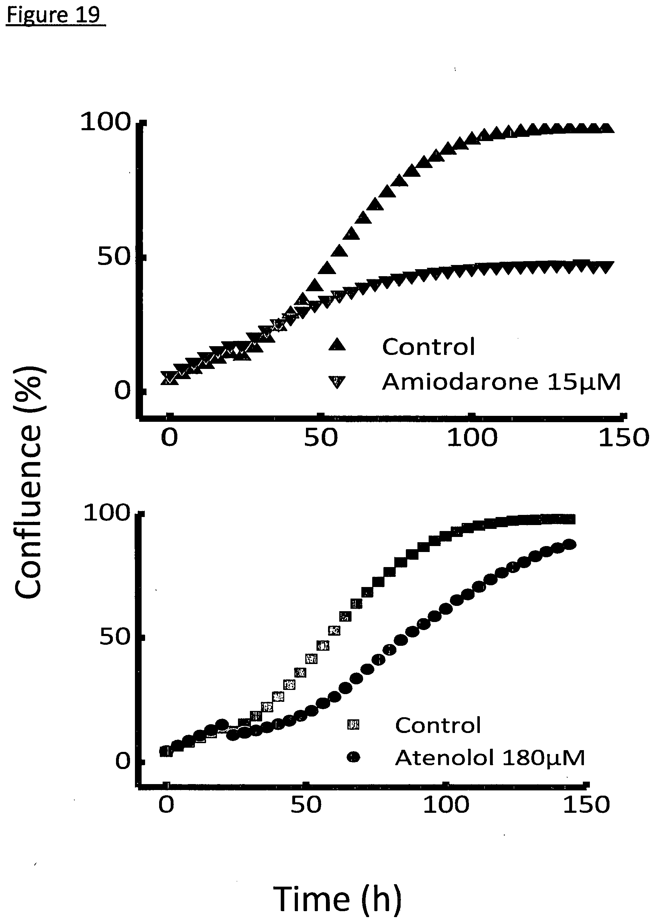

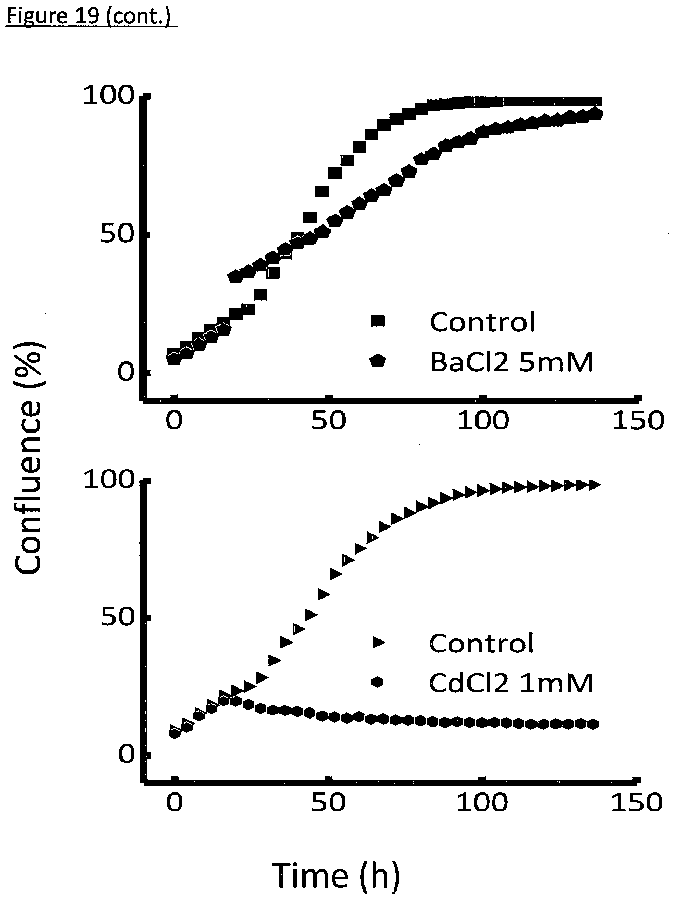

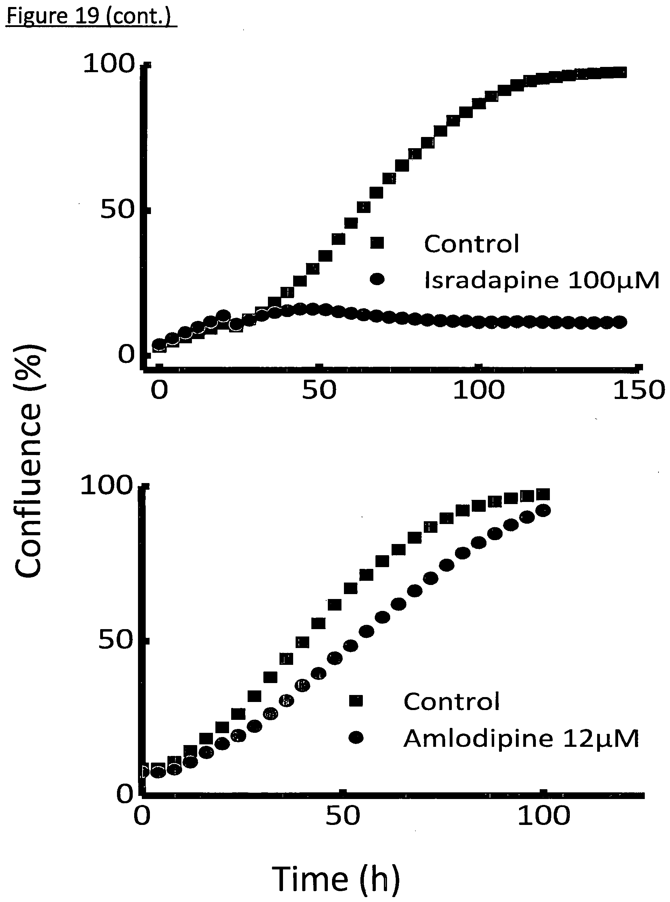

Figure 19 shows that Membrane Potential Regulating Compounds (MPRCs) that inhibit Wnt signaling also inhibit cell growth of PC3 prostate cancer cell line. Growth curve was monitored at 37°C in a CO₂ incubator by using time lapse imaging with Incucyte (Essen Biosciences) for up to 150h after plating the cells (t=0) at a density of 25-100k cells per well in a 6 well plate (Nunc). MPRCs were added at the stated concentrations 24 h after cell plating;

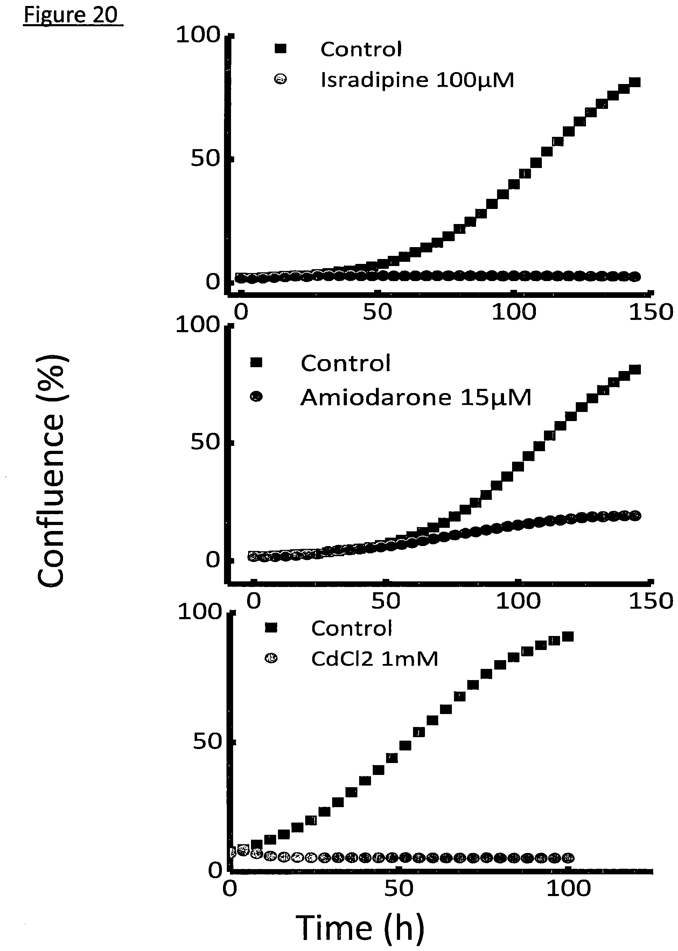

Figure 20 shows that Membrane Potential Regulating Compounds (MPRCs) that inhibit Wnt signaling also inhibit cell growth of MCF7 breast cancer cell line. Growth curve was monitored at 37°C in a CO₂ incubator by using time lapse imaging with Incucyte (Essen Biosciences) for up to 150h after plating the cells (t=0) at a density of 25-100k cells per well in a 6 well plate (Nunc). MPRCs were added at the stated concentrations 24 h after cell plating. Data for other MPRCs is given in Figure 12;

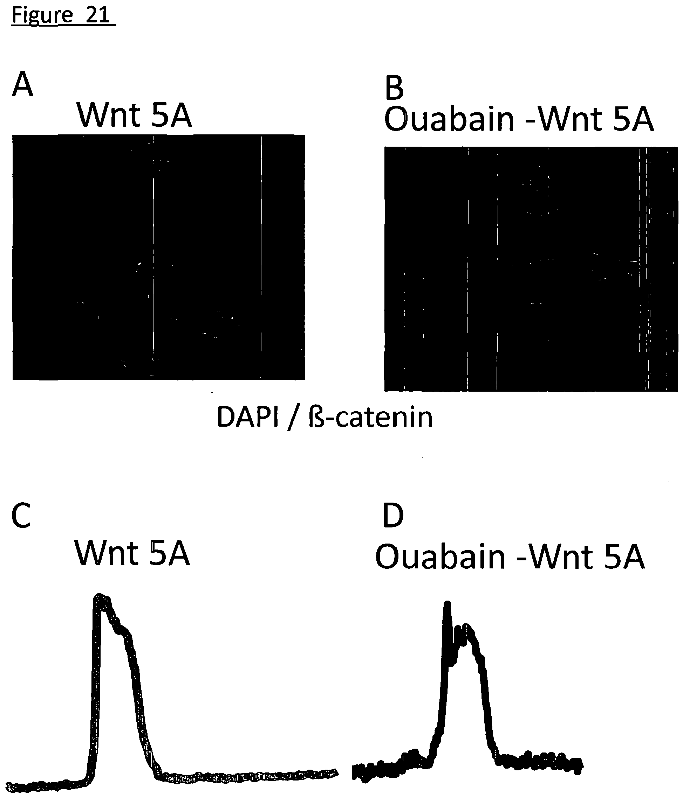

Figure 21 shows that Na/K ATPase inhibitor Ouabain increases β-catenin translocation to the nucleus. PC3 prostate cancer cells were treated with Ouabain and stained for β-catenin as described elsewhere (Thrasivoulou et al, 2013, J Biol Chem J Biol Chem 288: 35651-35659). (A) Wnt5A treated cells show translocation of
β-catenin (red) to the nucleus; (B) Cells pretreated with Ouabain show increased translocation of β-catenin (red); cell nucleus is counter-stained with DAPI. (C) Wnt induced calcium release in PC3 cells in response to addition of Wnt5A (example shown); (B) Wnt 5A induced calcium response after pretreatment with Ouabain; and Figure 22 shows that compounds such as Nicorandil and Veratradine do not inhibit Wnt signaling- measured using intracellular calcium release (A and B) or cell proliferation (C and D- nicorandil shown as an example). Wnt signaling calcium measurements were performed as described in (Thrasivoulou et al, 2013, J Biol Chem J Biol Chem 288: 35651-35659). Cell growth in PC3 (C) and MCF7 (D) were measured using Incucyte (Essen Bioscience).

### Reference Examples

### Materials and methods

### Cell lines

All cell lines (PC3, prostate cancer, MCF7, breast cancer, 253J bladder cancer and Cos 7 simian kidney) were obtained from ATCC via John R W Masters, University College London and cultured in RPMI 1640 (Invitrogen, Paisely, UK) medium containing 5mM L-glutamine and fetal bovine serum (FBS).

### Wnt peptides

Recombinant Wnt (3A, 4, 5A, 7A, 9B, 10B and 11) peptides were purchased from R&D systems (Minneapolis, MN). Wnt-peptide stock solutions were prepared in PBS.

### [Ca²⁺]ᵢ and membrane potential imaging

The imaging protocol was developed in the inventor's laboratory and has been described in detail, elsewhere (Wang at al., (2010) PLoS ONE 5, e10456). Briefly, 100000-200000 PC3, MCF7, 253J or Cos 7 cells were plated on 35mm FluoroDish (WPI) and grown for 48h; calcium indicators Fluo-4 (Invitrogen) and FuraRed (Invitrogen) at 1ng/ml were added to FluoroDish containing only 1ml of culture medium and incubated for 15-20 min at 37°C. The culture medium was removed and washed (x2) and replaced with 1ml PBS. Live cell calcium Imaging was performed on an Olympus FluoView FV 1000 confocal microscope (Olympus KeyMed) equipped with a 20x dry objective (numerical aperture = 0.75). Calcium indicators were excited with an argon laser line (488nm) and emissions recorded in the green channel (500 - 560nm) for Fluo-4 and red channel (600-700nm) for FuraRed after addition of vehicle control (PBS) or Wnt X (30-600ng/ml). Images were acquired every 1.1 or 2.2 seconds for up to 600-800s; image and data acquisition was performed using FluoView 1000 software (Olympus, UK). Fluorescent intensity was measured and data exported as a tab delimited file for further analysis using a spreadsheet. All imaging experiments were performed in duplicate; intracellular calcium at 100ng/ml was measured in at least 3-12 different passages (passage numbers 25 to 37) of cell line for each Wnt ligand. Wnt 11 was tested in at least 5 different passages at 3 different concentrations. To test if the Wnt activated calcium release was from [Ca²⁺]ᵢ stores, in some cases PC3 cells, loaded with calcium dyes, were incubated with 1-5µM of thapsigargin (Sigma, UK), resuspended in dimethyl sulfoxide (DMSO), for 20min at 37°C, prior to addition of Wnt ligand and live calcium imaging (as described above).

Experiments with the membrane potential sensitive dye FM4 64 (Invitrogen) and Fluo4 were performed in exactly the same manner as for the Fluo4/FuraRed but with different emission spectra detection for the FM4 64 indicator, i.e., excitation with 488nm for both indicators and emission detection at 500 ― 560nm for Fluo4 and 650 ― 800 for FM4 64. All other imaging/data acquisition conditions were the same as above.

The experiments were performed using 35mm, glass bottom, FluoroDish containing 1ml PBS, at 37°C, to which ligands were added at desired concentration. In a static, non-superfusing assay system there is a lag time before the start of a response, in this case an increase in the intensity of the Fluo4 signal due to the release of free calcium. To measure the diffusion constant under these conditions we used Pontamine Sky Blue (Sigma Aldridge), which fluoresces in the red spectrum with 488nm excitation, using standard practice for adding ligands; the diffusion constant was measured to be 23.3± 2.1/µm.s⁻¹ (means ± S.E.M, n=8). The calcium wave, in response to Wnt ligands, travelled across the FluoroDish.

### External K⁺ modulation and use of K⁺ channel inhibitors

To measure the effect of changes in membrane potential on [Ca²⁺]ᵢ release using the protocol described above, in some experiments, external solution (PBS) was substituted with varying concentrations of KCl. In other experiments chemical inhibitors of K+ channels such as TEA, CsCl or tolbutamide (Sigma-Aldrich) were added to the cells grown on FluoroDish, at 37°C, at least 5 min prior to addition of Wnt ligands.

### Data analysis

After image acquisition, regions of interest (ROI) were drawn over individual PC3 cells (up to 25) in a single frame and calcium intensity measurements were made using FluoView 1000 software (Olympus, UK). The measurements were exported as a tab delimited file into a spreadsheet for further analysis and Fluo4/Fura Red ratios were calculated. The waveform, a product of the ratio of Fluo4 and FuraRed over the period of measurement was used to measure the kinetics of the activation of [Ca²⁺]ᵢ release in PC3 cells, in response to different Wnt ligands. The time constants (rise, dwell and fall times) were calculated by first fitting the Fluo4/FuraRed ratio to a single Gaussian function (Origin Lab Corp., MA); a lower (10%) and upper (90%) amplitude thresholding method was then used to calculate the time constants (s). Statistical significance was calculated using Mann Whitney U test.

### Immunocytochemistry of mammalian cell lines

Immunostaining was performed in two different laboratories, independently with the experimenter blind to the identity of the treatment. Either cells used in FluoroDish, post [Ca²⁺]ᵢ experiments using dyes (see above) or grown in 8 well chamber glass slides (Lab Tek II, Nunc) were used. For Wnt ligand treatment, the culture medium replaced with PBS (without calcium) at 37°C. The experiments were replicated for Wnt ligand or vehicle control as described for [Ca²⁺]ᵢ measurements. Briefly, Wnt ligands were added and chamber slides incubated at 37°C for up to 20 min. Chamber wells were washed 3x with 0.5ml PBS and cells were fixed with 0.5ml 4% formalin for 30min at 4°C. Cells were washed 3x with ice cold PBS and stored in 0.5ml PBS at 4°C for immunostaining. In some experiments PC3 cells were treated with thaspsigargin as described above.

For immunostaining cells were washed twice for 5 minutes in TBST (20mM Tris pH 7.4, 0.9% NaCl, 0.1% Tween 20, Sigma). Cells were then incubated in 20% normal goat serum (NGS)/TBST for 30 min at room temperature, the chambers were drained and cells were incubated with mouse anti-β-catenin (ab22656, Abcam, UK) at 1:500 dilution in NGS/TBST overnight at 4°C. Following two further 5 min washes in TBST sections were incubated in goat anti-mouse peroxidase fab (Abcam, UK) at 1:500 dilution for 30 min at room temperature. Following a further two 5 min washes in TBST cells were incubated with tyramide Cy3 (Perkin Elmer) for 10 min at room temperature. Sections were washed twice for 5 min in TBST before nuclear counterstaining with DAPI. After a final two 5 min washes, the chambers were removed and cover slip placed on slides using Permafluor (Thermo Shandon).

### Imaging and quantitation forβ-catenin expression and nuclear co-localization analysis

Fluorescence immunocytochemical staining for β-catenin expression in cell lines was imaged using Olympus Fluoview 1000 Confocal Microscope (Olympus KeyMed) mounted on an Olympus IX81 inverted microscope using a 20× 0.7NA or 40× 1.0NA dry objective with a zoom of x3.4',6-diamidino-2-phenylindole (DAPI, nuclear counterstain) was excited at 405nm with an emission of 415 - 500nm; Cy3 (β-catenin label) signal was excited at 561nm with an emission spectrum between 570 - 670nm. Laser power, gain and off-set settings for the photomultipler tubes (PMT) were kept similar for comparable samples. Z-stacks of at least 3 fields per sample were imaged and saved in Olympus FV (OIF or OIB) file format.

Nuclear co-localization of β-catenin was calculated by importing FV files for untreated, control or treated cells, into colocalization plugin of Fiji software (http://fiji.sc/Fiji) for Cy3 (β-catenin) and DAPI (nuclear) signals. Pearson corelation of colocalization were tabulated for 25-90 cells (from 2-5 different passages of cells) using ROI analysis. Statistical significance between untreated and treated cells was calculated using Mann Whitney U test.

### Scratch wound assay

PC3 cells were grown in 24 well, ImageLock plates (Essen Bioscience, MI) to confluency and scratched using a wound maker device (Essen Bioscience, MI). Imaging (at 2h intervals) and quantitation of wound width was performed as described earlier (Wang at al., (2010) PLoS ONE 5, e10456) using Incucyte (Essen Bioscience, MI). The rate of wound closure (± Wnts and thapsigargin) was calculated by fitting the line using linear regression function in Origin software (Origin Lab Corp., MA). Statistical significance of difference between the slopes for untreated and treated samples was calculated using F-test.

### Cell proliferation assays

A live imaging system (Incucyte, EssenBiosciences) was used to measure the effect of K⁺ channel inhibitors on the rate of cell proliferation. PC3 cells were plated on 6 well plates (Nunc) in 3ml culture medium (Wang et al (2010) PLoS ONE 5, e10456); K⁺ channel inhibitors were added to the wells at 0-15mM. The plates were placed in Incucyte in a standard cell culture incubator at 37°C and CO₂. Each well was imaged at an interval of up to 4 h and rate of cell proliferation was calculated from these images using Incucyte software.

### Compounds and materials

The following compounds were used.

| **Compound** | **Supplier** | **Catalogue No.** |
|---|---|---|
| Ouabain | Sigma | 03125 |
| Nifedipine | Sigma | N7634 |
| Amlodipine | Sigma | A5605 |
| Atenolol | Sigma | A7655 |
| Veratridine | Sigma | V5474 |
| Propanaolol | Sigma | P8688 |
| CdCl₂ | Sigma | C-2544 |
| Isradipine | Sigma | I6658 |
| Minoxidil | Sigma | M4145 |
| Dofetilide | Sigma | PZ0016 |
| Amiodarone | Sigma | A8423 |
| Loxapine | Sigma | L106 |
| Nicorandil | Sigma | N3539 |
| Furosemide | Sigma | F4381 |

- Penile tissue arrays were purchases from US Biomax. Prostate tissue array was custom made in the lab.
- Antibodies were purchased from Abcam (UK), except cyclin D1 (purchased from Santa Cruz, US) and Comyc (Novacastra, UK).

### Example 1 - Activation of intracellular calcium by multiple Wnt ligands and translocation of β-catenin into the nucleus: a convergent model of Wnt/Ca²⁺ and Wnt/β-catenin pathways

The inventors hypothesized that a Wnt mediated increase in [Ca²⁺]ᵢ leads to an increase in the nucleoplasmic calcium and depolarization of NE that facilitates the passage of β-catenin across the NE. Identity of Wnts that can activate [Ca²⁺]ᵢ release in mammalian cells is not well-characterized. In prostate cancer cells Wnt5A induces a large increase in [Ca²⁺]ᵢ, but it is not known whether this causes an increased nucleoplasmic Ca²⁺ and depolarization of the NE, and if there is subsequent translocation of β-catenin to the nucleoplasm. In order to test their hypothesis, the inventors first determined (i) if Wnts other than Wnt5A also increase in [Ca²⁺]ᵢ (ii) that increase in [Ca²⁺]ᵢ due to Wnt/Ca²⁺ signaling activation results in depolarization of nuclear membranes (iii) that in cells, activated by multiple Wnt ligands, β-catenin is translocated into the nucleus. By using a live fluorochrome assay and subsequent assessment of β-catenin translocation to nucleoplasm in Wnt ligand activated mammalian cancer cell lines (PC3, prostate, MCF7, breast and 253J bladder cell lines), the inventors have demonstrated that a number of Wnt ligands increase intracellular and intranuclear Ca²⁺, with distinct kinetics, depolarization of the NE and translocation of β-catenin to the nucleus; both the increase in Wnt induced [Ca²⁺]ᵢ release and β-catenin translocation are suppressed by thapsigargin, an inhibitor of microsomal Ca²⁺-ATPase. A model in which Wnt/Ca²⁺ and Wnt/β-catenin pathways are coupled rather than independent, as previously thought, is also proposed.

### Results

The inventors have shown, previously, that Wnt/Ca²⁺ signaling is a key regulator of cytoskeleton and motility in PC3 cells with a 2.5 fold increase in [Ca²⁺]ᵢ (Wang et al (2010) PLoS ONE 5, e10456). Very little is known about the ability of Wnts, other than Wnt5A induces intracellular Ca²⁺ release in prostate and other mammalian cells. The first question the inventors sought to answer was which other Wnts induce [Ca²⁺]ᵢ release in PC3 cell lines?

### [Ca²⁺]ᵢ release in response to Wnt ligands

Addition of all Wnt ligands tested, with the exception of Wnt11, caused a change in ratio of Fluo4/Fura Red in PC 3 cells. The ratio of Fluo4/FuraRed was plotted as a function of time (Figure 1, representative trace for all Wnts tested) and the waveform was used to measure the kinetics of the activation of [Ca²⁺]ᵢ in PC3 cells, in response to different Wnt ligands. Parameters such as the amplitude and time constants (rise, dwell and fall times) were calculated by first fitting the Fluo4/FuraRed ratio to a single Gaussian function. The inventors then used a lower (10%) and upper (90%) amplitude thresholding method to calculate the timing parameters. The amplitude for [Ca²⁺]ᵢ release in response to Wnt ligands represents the peak of the calcium waveform. The amplitude of calcium release was Wnt ligand concentration dependent. A number of Wnt ligands (e.g. 5A, 9B and 10B) also activated [Ca²⁺]ᵢ release in MCF7 and 253J human breast and urinary tract cancer cell lines, respectively, indicating that this phenomenon was not specific to PC3 cell line. Furthermore, Wnt5A also activated [Ca²⁺]ᵢ in Cos7 simian kidney cell line.

Thapsigargin is an inhibitor of the endoplasmic reticulum Ca²⁺-ATPase and depletes [Ca²⁺]ᵢ stores (Thastrup, O. et al (1990) Proc. Natl. Acad. Sci. U.S.A 87, 2466-2470). Thapsigargin has been used, previously, to investigate ligand induced [Ca²⁺]ᵢ release. The increase in [Ca²⁺]ᵢ in response to Wnt ligands tested (5A, 9B and 10B) was abolished after treatment of PC3 cells with thapsigargin (5µM) (Figure 2). These results indicate that depletion of [Ca²⁺]ᵢ abolishes Wnt induced [Ca²⁺]ᵢ release.

### Rise, dwell and fall time of [Ca²⁺]ᵢ release

Time constants, for the rise, dwell and fall time of [Ca²⁺]ᵢ release in response to Wnt peptides, (100ng/ml) were calculated as described in experimental procedures and shown in Figure 3. Wnt3A and Wnt5A showed the shortest rise and fall time whereas Wnt 9B and Wnt4 showed the slowest rise and fall times (Figure 3A and C). DTs reflect the time the [Ca²⁺]ᵢ signal was sustained at peak amplitude after application of Wnt peptides. The DT analysis (Figure 3B) indicate that of the six Wnts activating [Ca²⁺]ᵢ release could be classified into 3 classes: 3A and 5A, short (DT<15s), 7A and 10B, long (DT>25s) and 4 and 9B, very long (DT>30s).

### Wnt ligands activate calcium by a negative co-operative mechanism

The kinetics of [Ca²⁺]ᵢ release was further analyzed by using three different concentrations for each Wnt ligand. Box-whisker plots for the rise, dwell and fall time as a function of low, medium and high concentrations of each Wnt ligand is given in. Time constants were significantly different between at least two different concentrations for Wnts 3A, 4, 5A, 9B and 10B but no difference in these time constants was observed for Wnt7A. The inventors compared the DT constants for Wnts 3A, 4, 5A, 9B and 10B in more detail to elucidate the mechanisms of [Ca²⁺]ᵢ release by fitting the DT data to calculate the reaction-rate expression for a firstorder reaction (Figure 4). Strikingly, Wnts 3A, 4, 9B and 10B show negative co-operativity but not Wnt5A. Negative co-operativity indicates a decrease in function (DT) as the ligand concentration increases; the rate constants for Wnts showing negative co-operativity are given in Figure 4. Wnt5A was the only [Ca²⁺]ᵢ activating Wnt ligand that follows the classical Michaelis-Menten kinetics, with a half-maximal concentration of 67ng/ml. These results provide the first evidence of negative co-operativity of binding of Wnts (3A, 4, 9B and 10B) to their receptor and show, also for the first time, that the molecular dynamics of [Ca²⁺]ᵢ release, via receptor/ligand interaction, by different Wnts, could provide critical regulatory mechanisms of Wnt/ Ca²⁺ signaling.

### [Ca²⁺]ᵢ release results in increased intranuclear Ca²⁺ and altered electrical activity in the nucleus

Calcium is a key regulator of the electrical activity in the nucleus (Mazzanti, M. at al (2001) Physiol Rev. 81, 1-19). Intranuclear concentration of Ca²⁺ follows increase in cytosolic Ca²⁺ (Allbritton et al (1994) Proc. Natl. Acad. Sci. U.S.A 91, 12458-12462). The inventors therefore investigated changes in intranuclear calcium and membrane potential using cells loaded with Fluo4 and FM4-64 with or without Wnts in PC3 and MCF cells. There was an increase in the [Ca²⁺]ᵢ (Fluo4) signal in the green channel (500 - 560nm) upon addition of the Wnt ligands in these cells. After ~300s there was an increase in the intensity of the FM4-64 signal in the red channel (650 ― 800nm); there was depolarization of the cell membrane, as expected (Figure 5). There was also an increase in the intensity of FM4-64 around the nucleus, indicating a depolarization of the transnuclear envelope potential in PC3 cells (Figure 5).

### Wnt/Ca²⁺ signaling and cell motility

One functional consequence of inhibition of downstream Wnt/Ca²⁺ signaling is a decrease in cell motility (Wang et al (2010) PLoS ONE 5, e10456). To test if inhibition of Wnt/Ca²⁺ signaling by Wnt/canonical signaling ligands such as Wnt9B and 10B, the inventors used the wound scratch assay in which Wnt induced [Ca²⁺]ᵢ release was inhibited by thapsigargin. The rate of wound closure in wounded PC3 cells in the presence of Wnt 5A or 9B was similar to that of control untreated cells. However, there was a significant decrease in the rate of wound closure in cells treated with thapsigargin even in the presence of Wnt ligands. These results indicate that downstream mechanisms of Wnt/Ca²⁺ signaling are similar for the classical non-canonical (Wnt 5A) or canonical (Wnt 9B) Wnt ligands. Similar results were obtained for Wnts 3A and 10B when [Ca²⁺]ᵢ was inhibited by incubation with thapsigargin.

### Translocation of β-catenin to the nucleus subsequent to nuclear membrane depolarization

β-catenin expression was determined subsequent to Wnt ligand activation and [Ca²⁺]ᵢ release using immunofluorescence. Compared to vehicle controls there was an increased in expression of β-catenin in PC3 cells treated with Wnts5A, 9B and 10B (Figure 6). There was little expression of β-catenin in vehicle treated cells (Figure 6A); the expression of β-catenin is visibly increased in PC3 cells treated with Wnt5A (Figure 6B), Wnt9B (Figure 6C) and Wnt 10B (Figure 6D) and is visible in the nucleus. Similar results were obtained for Wnt 3A and Wnt4 in PC3 cells. Colocalization of β-catenin and DAPI signals was quantified using Fiji software (Schindelin et al (2012) Nat.Methods 9, 676-682) as described in methods. There was a significant increase in the Pearson coefficient of nuclear co-localization of β-catenin when cells were exposed to Wnts 3A, 4, 5A, 9B and 10B (Figure 7). Furthermore, inhibition of Wnt induced [Ca²⁺]ᵢ release, by incubation of cells with thapsigargin, reduced the translocation of β-catenin into the nucleus, indicating a link between β-catenin translocation, store operated, thapsigargin inhibitable [Ca²⁺]ᵢ release and perhaps alterations in nuclear envelope potential.

The inventors also used the paradigm ligands of the Wnt classes (as categorized in Figure 3) in two other cell lines to determine that an increase in [Ca²⁺]ᵢ release was followed by translocation of β-catenin to the nucleus. The results indicate that increase in [Ca²⁺]ᵢ in response to a variety of Wnt ligands leads to a depolarization of the nuclear envelope and β-catenin translocation from the cytosol to the nucleus.

### Discussion

Wnt signaling is categorized into, independent, canonical and non-canonical pathways, with β-catenin a key transducer of the former and Ca²⁺ a major effector molecule for the latter (other non-canonical pathways include the planar cell polarity (Gubb et al (1982) J.Embryol.Exp.Morphol. 68, 37-57; Hammerschmidt et al (1996) Development 123, 143-151) and cGMP/PDE pathways. Wnt/β-catenin pathway is used as a synonym for the canonical pathway. The categorization is not absolute as recent examples show some cross talk, however, the distinction of β-catenin dependent and independent pathways to describe Wnt signaling remains unresolved. The inventors have addressed two key questions regarding Wnt signaling in mammalian cells:- (1) Which and how many Wnt ligands can mobilize intracellular calcium?; and (2) What are the mechanisms of β-catenin entry into the nucleus? The data show, for the first time, that in mammalian cells, six out of the seven Wnts tested in a direct calcium measurement assay mobilize [Ca²⁺]ᵢ, including Wnt ligands previously thought to act only via β-catenin pathway (e.g. Wnt 9A, 10B) (Figure 1). A discharge of [Ca²⁺]ᵢ from endoplasmic reticulum and inhibition of microsomal Ca²⁺-ATPase by incubation of cells with thapsigargin abolished the increase in Wnt induced [Ca²⁺]ᵢ release (Figure 2) and reduced β-catenin translocation to the nucleus. Thapsigargin is resuspended in dimethyl sulfoxide (DMSO). DMSO induces Ca²⁺ spikes in a variety of cells; the results in PC3 cells concur with previous observations. Thapsigargin also induced Ca²⁺ transient which declined to reach the baseline within 120 s (Figure 2). However, 15 min incubation with DMSO, had no effect on Wnt induced increase in [Ca²⁺]_{i,}, unlike thapsigargin treatment which abolished Wnt induced [Ca²⁺]ᵢ, release (Figure 2), indicating that Wnts activates store operated Ca²⁺ release.

The results provide first ever evidence that key transducers of canonical and non-canonical pathways converge to activate Wnt signaling in mammalian cells. A new convergent model of Wnt signaling network is proposed (Figure 8) in which multiple Wnt ligands in a variety of mammalian cell lines, increase free intracellular and nucleoplasmic Ca²⁺, depolarize the NE and increase the translocation of β-catenin to the nucleoplasm, suggesting a coupled rather than independent Wnt/Ca²⁺ and β-catenin pathway. Proposed steps of Wnt signaling in mammalian cells: [1] binding of Wnts (3A, 4, 5A, 7, 9B and 10B) results in [2] the activation of intracellular calcium stores that [3] increase in the intracellular concentration of free store operated calcium (Figures 1, 2 and 3). The increase in free calcium depolarizes the cell membrane and as [4] calcium enters the nucleus, the NE is depolarized (Figure 5); the calculated diffusion constants for the activation of bound to free intracellular calcium is 0.21µm / s and time constants for steps 1 to 4 are likely to be in milli-seconds.

The inventors have shown, previously, that activation of Wnt/Ca²⁺ pathway by Wnt5A, increases [5] CamKII activity to induce cytoskeletal modulation in prostate cancer cells (Wang at al., (2010) PLoS ONE 5, e10456). It is well established by previous studies (see Nusse, R. (2012) Cold Spring Harb. Perspect. Biol. 4, pii: a011163 for a review) that activation of Wnt signaling [6] desequesters the caged β-catenin (that is phosphorylated (P) and ubiquitinated (UUU) prior to degradation in proteasomes, gray square), in the cytosol. The desequestered β-catenin [7] translocation is facilitated across the NE (Figure 5), which is now [8] depolarized and has increased nucleoplasmic calcium. [9] β-catenin is now co-localized in the nucleus (Figures 6 and 7) where it is known to bind to LEF/TCF proteins to initiate gene transcription. For simplification, many other proteins involved in Wnt signaling are not shown, nor are multiple other pathways, such as Wnt/PCP pathways are included in the illustration. The steps for this evidence are shown with arrows shaded purple.

### Categorization of Wnt ligands and distinction into canonical and non-canonical pathways

The classification of Wnt ligands mediated signaling via the β-catenin or calcium pathways is largely based upon their ability to induce a secondary axis in *Xenopus* embryos or their ability to transform C57mg cells. Ecotopic expression of Wnts such as Wnt1, 3A, 8 and 8B was found to induce a secondary axis in *Xenopus* embryo and stabilize β-catenin and other Wnts such as Wnt5A and 11 did not. Members of the Wnt1 class include Wnt1, Wnt2, Wnt3, Wnt3A, Wnt8, Wnt8B and Wnt10B (Wnt1 shares 96% identity in amino acid sequence to Wnt10B and overlap in function) and induce secondary body axis in *Xenopus* and activates the canonical Wnt/β-catenin pathway. The Wnt5A class cannot induce secondary body axis formation in *Xenopus* and includes Wnt4, Wnt5A, Wnt5B, Wnt6, Wnt7A and Wnt11. Studies in Zebra fish embryos indicate that Wnt5A and Wnt11 but not Wnt8 over-expression induce calcium release.

However, these distinctions have been blurred when it was shown that Wnts associated with the so called non-canonical pathway are capable of transforming cells in culture. In addition, the ligands of Wnt family are likely to share the cell membrane receptor and most cells are exposed to a variety of Wnt ligands, simultaneously. A balance of these signals and the pathways they might activate (e.g. Wnt/β-catenin or Wnt/Ca²⁺ or both) could determine, for example, cell fate of a stem cell or whether cancer cells metastasize (e.g. by acquiring increased capacity for motility). Very little is known about the ability of Wnts, other than Wnt5A, to induce intracellular Ca²⁺ release in prostate or other mammalian cells. Wnt 11 is the only ligand tested in our study that did not mobilize [Ca²⁺]ᵢ. A possible explanation for this may reside in primary amino acid (AA) that deviates from the consensus Wnt sequence in 5 AA residues, in close proximity to RWNC motif.

### β-catenin translocation into nucleus subsequent to increase nucleoplasmic Ca²⁺ and depolarization of the NE

Macromolecular transport across the NE is comprised of a variety of independent and inter-dependent biochemical and electrochemical processes. These may involve a NLS binding of proteins intended for the nucleus with other cytosolic proteins to facilitate their entry requiring energy dependent mechanisms or phosphorylation of specific proteins. Although β-catenin does not contain NLS, by using *in vitro,* biochemical experiments, Sharma and colleagues have shown that this recognition may occur by the interaction of the Arm domain of β-catenin with NPC component proteins. Even when it reaches the outer NE, β-catenin faces considerable barriers to entry into the nucleoplasm and these experiments that describe the targeting of β-catenin to the outer NE but do not explain how β-catenin, a 92kDa, negatively charged protein (-20.8 at pH 7.0) may overcome significant biophysical hurdles to traverse the NE in response Wnt-signaling in cells.

Other mechanisms involve silencing of NPC channel activity for transport of medium sized (~40kDa) transcription factors (such as NFκB or SP1). Ca²⁺ ions also play a role in the trans-NE transport of macromolecules and it has been proposed that depletion of intranuclear Ca²⁺ can inhibit macromolecular transport of particularly intermediate size (70kDa) but also small (<10kDa) macromolecules across NE. Translocation of β-catenin, a 92kDa transcription factor, from the cytosol to the nucleus is a key event in the so-called canonical Wnt signaling, however the mechanism of this translocation is not known. The increase in intranuclear Ca²⁺ and depolarization of the nuclear membrane (Figure 5) and reduction in β-catenin translocation into the nucleus in the presence of thapsigargin suggest a putative role of membrane potential regulating ions as regulators of Wnt signaling in PC3 cells.

In the convergent model of Wnt signaling in mammalian cells, proposed here (Figure 8), binding of a variety of Wnt ligands to their receptors activates [Ca²⁺]ᵢ release (Figure 1). The sharp and transient increase in [Ca²⁺]ᵢ, results in the increased intranuclear Ca²⁺ within 300s of Wnt ligand activation (Figure 5). Subsequent to intranuclear Ca²⁺ increase and depolarization of the NE, there is also an evident increase in the nuclear expression of β-catenin (Figures 6 and 7). The model presented here does not exclude upstream homing events that may direct β-catenin to the NE or downstream events, such as involvement of energy expenditure or other biochemical and macromolecular transport mechanisms that may also facilitate the ultimate translocation of β-catenin into the nucleus. It however includes an important and as yet unconsidered electrochemical aspect in the transport of β-catenin into the nucleus where its functional activity lies. It also suggests, at least in mammalian cell lines, an integrated signaling process that can facilitate, amplify and regulate activities of multiple transducers activated by individual ligands (Figure 8).

### Example 2 - Therapeutic use of regulating membrane potential and intracellular calcium to control diseases in which β-catenin translocation to the nucleus is dysregulated

As described in Example 1, the inventors have provided a new convergent model of Wnt signaling in which [Ca²⁺]ᵢ release facilitates β-catenin entry into the nucleus.

### Results

The inventors have previously shown in Example 1 that release of [Ca²⁺]ᵢ, prior to stabilization of β-catenin, facilitates β-catenin translocation to the nucleus. Release of [Ca²⁺]ᵢ (or an increase in intracellular K+ or Na⁺) in addition to depolarizing the cell membrane also depolarizes the intracellular membranes, including the nuclear envelope Mazzanti, M., et al (2001) Physiol Rev. 81, 1-19 . K⁺ concentration gradient, along with that of Na+, across the cell membranes is a key regulator of membrane potential, with intracellular Ca2+ also playing a role. A decrease in the K⁺ concentration gradient (e.g. by a small increase in extracellular K⁺ concentration) would depolarize the cell membrane and an increase in the prevailing K+ gradient would hyperpolarize the membrane potential (both depolarization and hyperpolarization are relative terms). The first question the inventors asked was if altering external K+ concentration would alter Wnt induced release of [Ca²⁺]ᵢ ?

### Changes in membrane potential and [Ca²⁺]ᵢ release in response to Wnt ligands

To measure the effect of altered membrane potential on the rate of Wnt induce [Ca²⁺]ᵢ release, three different concentrations of K⁺ were used in the extracellular solution (Figure 9). Increasing extracellular K+ and thereby decreasing trans-cellular K⁺ gradient inhibited Wnt induced [Ca²⁺]ᵢ release (Figure 9), indicating that Wnt signaling was electrogenic. This was a novel observation. β-catenin, the second transducer of Wnt signaling was also dependent upon the prevailing membrane potential, as decrease in K+ gradient increased Wnt-mediated β-catenin translocation to the nucleus (Figure 10). Although the latter may appear counterintuitive, at first sight, the depolarization (cell membrane positive inside compared to outside) will have the same outcome as an increase in intracellular positive charges due to release and transport of free Wnt mediated [Ca²⁺]ᵢ release, particularly on the nuclear membrane potential. This observation was confirmed with a membrane potential dye.

### K+ channel blockers and Wnt mediated [Ca²⁺]ᵢ release, β-catenin stabilization and translocation to the nucleus

Having established the role of membrane potential, particularly Ca²⁺ and K+ ions, in Wnt signaling , the inventors next tested if blocking K+ channels would alter Wnt signaling , particularly β-catenin translocation to the nucleus. To do this they incubated PC₃ cells with a range of known K+ channel blockers. Pre-incubation of PC₃ cells with TEA (a general K+ channel blocker) reduced the Wnt induced [Ca²⁺]ᵢ release (Figure 11). Furthermore, incubation of PC₃ cells with TEA and other K⁺ channel inhibitors such as CsCl (a blocker of Kir 1 type channels) and tolbutamide (blocks Kir 6 type channels), all within physiological concentrations, reduced the Wnt mediated β-catenin stabilization and translocation to the nucleus (Fig 11B); the general inhibitor of K+ channels, TEA, showed the biggest reduction in Wnt mediated stabilization and translocation of β-catenin.

### K+ channel blockers and cell proliferation

The role for β-catenin proliferative diseases such as cancer is well established (Grant et al (2006) Nat. Genet. 38, 320-323). If K⁺ channel inhibitors reduce Wnt mediated β-catenin translocation to the nucleus, it follows that there would be a decrease in cell proliferation when cells are incubated with these inhibitors. The inventors measured rate of cell proliferation by using cell growth curves using Incucyte (see materials and methods). Addition of all K+ and Ca²+channel inhibitors caused a decrease in the rate of cell proliferation (Figure 12). These results indicate that inhibition of K+ channels in PC₃ cell line inhibits cell growth, possibly by reducing or inhibiting Wnt signaling.

### Discussion

The data presented herein demonstrates that: (i) modulation of membrane potential or (ii) blockade of channels (e.g. with TEA, tolbutamide) inhibits Wnt mediated [Ca²⁺]i release and translocation of β-catenin to the nucleus and (iii) decreases cell proliferation. Wnt mediated translocation of β-catenin to the cell nucleus is a key event in diseases as varied as cancer or osteoporosis. It has been long thought that regulating β-catenin translocation to the nucleus by targeting cell membrane protein(s) could be a useful approach to counter these diseases. However, classical pharmacological approaches have not yielded useful or effective therapies and have generally proved difficult.

The inventors' approach, discovered due to the formulation of a new convergent model of Wnt signaling discussed in Example 1, indicates that Ca²⁺ and the prevailing cell and nuclear membrane potential are key regulators Wnt signaling. They have also shown that this occurs in cell lines from different organs (e.g. prostate, breast, bladder, kidney) and is likely to be a universal cellular phenomenon. Membrane potential regulation is a key cellular process that is largely conducted by the activities of ion transporters in the membranes. The results show, clearly, that altering membrane potential or intracellular Ca²⁺ release, modulates β-catenin translocation to the nucleus. This opens up a new avenue for targeting the Wnt signaling pathway with compounds and regulators that have been in scientific research and clinical use for many decades. Unlike downstream, cytosolic regulators of Wnt signaling (e.g. kinases, such as GSK3β, inhibitors), most cell membrane potential regulators are integral cell membrane proteins accessibility of drug(s) in *vivo* does not pose a hurdle.

From the data presented here, it appears that altering the membrane potential by dissipating trans-cellular K+ gradient, or by inhibiting K+ channels with known inhibitors, alter both the Wnt mediated [Ca²⁺]ᵢ release and β-catenin translocation. The mechanism of this reduction in Wnt signaling appears to be both through alterations in [Ca²⁺]ᵢ, changes in cell membrane and nuclear membrane potential (Figures 9-11). This means that it will be possible to use a variety of compounds such as those that depolarize the membrane potential (e.g. Na+ channel openers such as BTL or Loxapine) to increase Wnt mediated β-catenin translocation membrane for diseases, such as osteoporosis, where there is a reduction of β-catenin translocation to the nucleus (Baron, R. and Kneissel, M. (2013) Nat. Med. 19, 179-192).

Conversely, in diseases such as cancer there is an increase in Wnt signaling and consequent increase of β-catenin to the nucleus (Clevers, H. and Nusse, R. (2012) Cell 149, 1192-1205; Polakis, P. (2007) Curr. Opin. Genet. Dev. 17,45-51; Polakis, P. (2012) EMBOJ. 31, 2737-2746). The results show that a number of channel inhibitors (see Figures 10-12) reduce both Wnt mediated [Ca²⁺]ᵢ release and reduce cell proliferation, indicating that inhibitors of K+ channels, independently or by reducing Wnt mediated [Ca²⁺]ᵢ release, could reduce β-catenin translocation to the nucleus. Indeed electrophysiological investigations also support the idea that Wnt signaling is electrogenic and is regulated by both prevailing membrane potential and Ca²⁺ regulators (Ahmed, A and Olsen, H, unpublished data).

The novel use of drugs such as CsCl, TEA, tolbutamide and others (see Table 1) is proposed as a therapy for prostate, breast, colon (Baron, R. and Kneissel, M. (2013) Nat. Med. 19, 179-192) and pituitary and other cancers where Wnt mediated β-catenin translocation is involved in carcinogenesis. It is also evident that depolarizing membrane potential (e.g. by increasing inward movement of Na+ with channel openers such as BTL or Loxapine) will also be an effective therapy for osteoporosis where there is a clear role for β-catenin in differentiation of osteoblasts to osteoclasts. Tolbutamide (drug name Orinase) has been known to stimulate insulin release through its action on K_{ATP} channels. The inventors observed an inhibition of β-catenin translocation (Figure 11) and cell proliferation (Figure 12) in the presence of tolbutamide. Given the role of β-catenin/TCF/LEF mediated gene transcription, inhibiting β-catenin translocation by other molecules or antibodies could also be used as a therapy for diabetes (type II in particular).

The rationale that inhibition of Wnt mediated [Ca²⁺]ᵢ release and reduction of β-catenin translocation to the nucleus and transcription is likely to occur in cancer is *indirectly* supported by published epidemiological studies: Beta blockers are a standard treatment modality for hypertension and act via increased cyclic AMP and increase in activity of calcium dependent calmodulin kinase (a downstream target of Wnt and other calcium signaling pathways). It is also known that treatment for reduction in blood pressure with beta blockers is accompanied by reduction in free intracellular calcium (Baumgart et al (1986) J. Cardiovasc. Pharmacol. 8, 559-561). A decrease in free intracellular calcium will result in hyperpolarization of cell and nuclear membrane potential (Geiszt, M. et al (1997) J. Biol. Chem. 272, 26471-26478; Rada et al (2004) Blood 104, 2947-2953). It has been suggested in three independent epidemiological studies in breast, skin and prostate cancers that patients on beta blockers have a lower incidence of cancer progression and mortality (Barron et al (2011) J. Clin. Oncol. 29, 2635-2644; Melhem-Bertrandt et al (2011) J. Clin. Oncol. 29, 2645-2652; Lemeshow et al (2011) Cancer Epidemiol. Biomarkers Prev. 20, 2273-2279; Grytli et al (2013) Eur. Urol.*).*

Table 1 below lists a number of compounds which can be used to treat β-catenin related diseases, such as cancer, diabetes and osteoporosis.

**Table 1: Drugs and compounds proposed for the treatment of β-catenin related diseases, such as cancer, diabetes and osteoporosis**

| **Compound** | **Dose (mg/day)**^{∗} | **1st medical use** | **and use** | **Route** | **Modulates** |
|---|---|---|---|---|---|
| Dofetilide (Tykosin) | 500 | CdAr | Ca/Db | Oral | K-channel |
| Etamon | 400-500 | CdAr | Ca/Db | i.v. | K-channel |
| CsCl | 1000-5000 | CdAr | Ca/Db | Oral | K-channel |
| Sotalol | 50-150 | CdAr | Ca/Db | Oral | K-channel |
| Amiodarone | 240 | CdAr | Ca/Db | i.v. | K-channel |
| 4-aminopyridine (Ampyra) | 20 | Hypertension | Ca/Db | Oral | K-channel |
| Pinacidil | 30 | Hypertension | Ca/Db | Oral | K-channel |
| Nicorandil | 10-40 | Angina | Ca/Db | Oral | K-channel |
| Glibenclamide (Micronase) | 1.25 | Diabetes | Ca | Oral | K-channel |
| Tolbutamide¶ | 2500-3000 | Diabetes | Ca | Oral | K-channel |
| Tolazimide | 100-250 | Diabetes | Ca | Oral | K-channel |
| Minoxidil | 10 | Hypertension | Ca/Db | Oral | K-channel |
| Quinidine | 100-600 | CdAr | Ca/Db | Oral | Na-channel |
| Lidocaine | 100 | CdAr/Anaesthetic | Ca/Db | i.v. | Na-channel |
| Bretylium tosylate | 350 | CdAr | Os | i.v. | Na-channel |
| Loxapine | 20-50 | Schizophrenia | Os | i.m. | Na-channel |
| Veratridine | NCCU | | Os | | Na-channel |
| Nifidipine/Adalat | 30-60 | Angina | Ca/Db | Oral | Ca-channel |
| Dihydropyridine | 2.5-10 | Hypertension | Ca/Db | Oral | Ca-channel |
| Bay K8644 | NCCU | | Ca/Db | | Ca-channel |
| FPL 64176 | NCCU | | Ca/Db | | Ca-channel |
| Isradipine | 5 | Hypertension | Ca | Oral | Ca-channel |
| Isatroxime (PST2744) | 12 | Cardiac failure | Ca/Db | i.v. | Na/KATPase |
| Digoxin | 0.5 | Cardiac failure | Ca/Db | Oral | Na/KATPase |
| Furosemide | 600 or 400(iv) | Hypertension | Ca | Oral / i. v. | Na-K-Cl transporter |

| | | | | | |
|---|---|---|---|---|---|
| *dosage for an average 70kg person over a 24h period ¶ other analogs: Orinase, Chlorpopamide § other analogs: Amlodipine, Isradipine, Larcanidipine CdAr = Cardiac Arrythmia; Ca = Cancer; Db= Diabetes; Os=Osteoporosis NCCU= not in current clinical use | | | | | |

Most of the proposed compounds that modulate the activity of an ion channel or transporter are in current clinical use for indications (1^{st} use) other than cancer or osteoporosis. These drugs and compounds are proposed for 2^{nd} use as a therapy for cancer, diabetes or osteoporosis.

### Targeted immunological therapy for cancer: -

### 1) Therapeutic antibodies that block tissue specific channels

### Example 3 - Prostate and Penile cancer

The inventors investigated Wnt signaling, and confirmed that it is increased in prostate and penile cancers, as shown in Figures 13, 14 and 15.

Figure 13 shows that Wnt signaling proteins are upregulated in prostate cancer tissue. There is a visible and quantitative difference in the expression of Wnt signaling targets in normal compared to cancerous prostate samples. Figure 14 shows that Wnt signaling proteins are up-regulated in human penile squamous cell carcinoma. Quantitative analysis was performed on the non-enhanced, original, gray images for which all settings were identical in all tissue core images analyzed. Figure 15 is a box plot of the quantitation of expression of WNT₄, MMP₇, CD₁ and c-MYC in PeCa tissue classified according to pathological grade. All proteins showed a significant difference in expression between control and malignant tumor samples.

### Example 4 ― Wnt signaling induces calcium release

Referring to Figure 16, there are shown the results of a series of membrane potential regulating compounds (MPRCs) which inhibit Wnt signaling as measured by Wnt induced calcium release. Figure 16A shows a representative trace of Wnt induced calcium release. The Wnt ligand induced calcium release was inhibited after preincubation for 5 min with MPRCs (CsCl, CdCl₂, Tetraethyl ammonium (TEA), Amiodarone, Tolbutamide, Isradipine, Nifedipine, Minoxidil, Atenolol, Propanolol, and Furosemide.

### Example 5 ― Wnt mediated β-catenin translocation

Referring to Figure 17, there is shown the results of various membrane Potential Regulating Compounds (MPRCs) which inhibit Wnt signaling as measured by Wnt mediated β-catenin translocation in PC₃ prostate cancer cell line. Cells were treated with Wnts with or without preincubation with MPRCs at 37°C in a CO₂ incubator. Wnts were added and cells were incubated for a further 20-30min, washed, fixed and stained.

Figure 18 shows that membrane Potential Regulating Compounds (MPRCs) inhibit Wnt signaling as measured by Wnt mediated β-catenin translocation in MCF 7 breast cancer cell line.

### Example 6 ― Prostate and breast cancer cell lines

Figure 19 shows that membrane potential regulation compounds (MPRCs) that inhibit Wnt signaling also inhibit cell growth of PC₃ prostate cancer cell line. Figure 20 shows that membrane potential regulation compounds (MPRCs) that inhibit Wnt signaling also inhibit cell growth of MCF7 breast cancer cell line.

### Example 7 -Ouabain increases Wnt β-catenin translocation to the nucleus

Referring to Figure 21, it shows that Na/KATPase inhibitor Ouabain increases β-catenin translocation to the nucleus. PC₃ prostate cancer cells were treated with Ouabain and stained for β-catenin.

### Example 8 - MPRCs activate Wnt signaling calcium measurement

Figure 22 shows that compounds such as Nicorandil and Veratradine do not inhibit Wnt signaling measured using intracellular calcium release (A and B) or cell proliferation (C and D- nicorandil shown as an example).

## Claims

1. A cell membrane electrical potential-regulating agent comprising Dofetilide, for use in treating a β-catenin-related disease, wherein the cell membrane electrical potential-regulating agent is arranged to decrease beta-catenin translocation to the cell's nucleus, and is used to treat prostate cancer.

2. An agent for use according to claim 1, wherein the agent is capable of blocking ATPase or an ion transporter or channel, which results in the regulation of the electrical potential of the cell membrane, and decreases β-catenin translocation to the nucleus.

3. An agent for use according to claim 2, wherein the ion channel or transporter, which is blocked, is a Na+, K+, Cl- or Ca²⁺ channel.

4. An agent for use according to any preceding claim, wherein the agent comprises an antibody.

5. An agent for use according to any preceding claim, wherein the cell membrane electrical potential-regulating agent, which modulates β-catenin translocation in the cell, is for treating a subject who is already being treated with a beta blocker.

6. A β-catenin-related disease treatment composition, comprising Dofetilide for use according to any preceding claim, which modulates β-catenin translocation in the cell, and a pharmaceutically acceptable vehicle.

## Patentansprüche

1. Mittel zur Regulierung des elektrischen Potentials einer Zellmembran, umfassend Dofetilid, zur Verwendung beim Behandeln einer mit β-Catenin zusammenhängenden Krankheit, wobei das Mittel zur Regulierung des elektrischen Potentials einer Zellmembran so angeordnet ist, um beta-Catenin-Translokation zu dem Zellkern zu verringern und verwendet wird, um Prostatakrebs zu behandeln.

2. Mittel zur Verwendung nach Anspruch 1, wobei das Mittel in der Lage ist, ATPase oder einen Ionentransporter oder -kanal zu blockieren, was in der Regulierung des elektrischen Potentials der Zellmembran resultiert und β-Catenin-Translokation zu dem Zellkern verringert.

3. Mittel zur Verwendung nach Anspruch 2, wobei der Ionenkanal oder Transporter, der blockiert ist, ein Na⁺-, K⁺-, Cl⁻- oder Ca²⁺-Kanal ist.

4. Mittel zur Verwendung nach einem vorhergehenden Anspruch, wobei das Mittel einen Antikörper umfasst.

5. Mittel zur Verwendung nach einem vorhergehenden Anspruch, wobei das Mittel zur Regulierung des elektrischen Potentials einer Zellmembran, das β-Catenin-Translokation in der Zelle moduliert, einem Behandeln eines Subjekts dient, das bereits mit einem Betablocker behandelt wird.

6. Zusammensetzung zur Behandlung einer mit β-Catenin zusammenhängenden Krankheit, umfassend Dofetilid, zur Verwendung nach einem vorhergehenden Anspruch, welche β-Catenin-Translokation in der Zelle moduliert, und ein pharmazeutisch verträgliches Vehikel.

## Revendications

1. Agent régulateur de potentiel électrique de membrane cellulaire comprenant du dofétilide, pour une utilisation dans le traitement d'une maladie liée à la β-caténine, ledit agent régulateur de potentiel électrique de membrane cellulaire étant conçu pour diminuer la translocation de la bêta-caténine vers le noyau de la cellule et étant utilisé pour traiter le cancer de la prostate.

2. Agent pour une utilisation selon la revendication 1, ledit agent étant capable de bloquer l'ATPase ou un transporteur ou canal d'ions, qui conduit à la régulation du potentiel électrique de la membrane cellulaire et diminue la translocation de la β-caténine vers le noyau.

3. Agent pour une utilisation selon la revendication 2, ledit transporteur ou canal d'ions, qui est bloqué, étant un canal d'ions Na⁺, K⁺, Cl⁻ ou Ca²⁺.

4. Agent pour une utilisation selon l'une quelconque des revendications précédentes, ledit agent comprenant un anticorps.

5. Agent pour une utilisation selon l'une quelconque des revendications précédentes, ledit agent régulateur de potentiel électrique de membrane cellulaire, qui module la translocation de la β-caténine dans la cellule, étant destiné à traiter un sujet qui est déjà traité avec un bêta-bloquant.

6. Composition de traitement d'une maladie liée à la β-caténine, comprenant du dofétilide, pour une utilisation selon l'une quelconque des revendications précédentes, qui module la translocation de la β-caténine dans la cellule, et un véhicule pharmaceutiquement acceptable.
